# EUROPEAN PATENT SPECIFICATION

(11) **EP 3 986 530 B1**
(45) Date of publication and mention of the grant of the patent: **25.11.2026**
(21) Application number: 20825818.6
(22) Date of filing: 26.05.2020
(51) Int. Cl.: G16H 40/67, G16H 20/00, A61N 1/00, H04M 1/725, H04W 4/00, A61N 1/372

(54) **SYSTEM, METHOD, AND ARCHITECTURE FOR FACILITATING REMOTE PATIENT CARE**
SYSTEM, VERFAHREN UND ARCHITEKTUR ZUR FERNBETREUUNG EINES PATIENTEN
SYSTÈME, PROCÉDÉ ET ARCHITECTURE PERMETTANT DE FACILITER LES SOINS AUX PATIENTS À DISTANCE

(30) Priority: 21.06.2019 US 201916449056
(43) Date of publication of application: 27.04.2022
(73) Proprietor: Advanced Neuromodulation Systems, Inc., Plano, TX 75024 (US)
(72) Inventor: IBARROLA, Germinal, Prosper, TX 75078 (US); DeBATES, Scott, Frisco, TX 75035 (US)
(74) Representative: Mathys & Squire
(86) International application number: PCT/US2020/034519
(87) International publication number: WO 2020/256894

(56) References cited:
- EP-B1- 2 185 065
- WO-A1-2017/165717
- WO-A2-2019/032788
- US-A1- 2002 082 665
- US-A1- 2002 082 665
- US-A1- 2011 171 905
- US-A1- 2015 089 590
- US-A1- 2017 111 488
- US-A1- 2017 325 091
- US-B1- 9 215 075
- US-B1- 9 288 614
- KIM HYUN, JO HYEONG, KANG SOON: "Self-Organizing Peer-To-Peer Middleware for Healthcare Monitoring in Real-Time", SENSORS (BASEL)., vol. 17, no. 11, 17 November 2017 (2017-11-17), pages 2650, 1 - 19, XP055776904, Retrieved from the Internet <URL:https://www.ncbi.nlm.nih.gov/pmc/articles/PMC5713001> [retrieved on 20200802], DOI: 10.3390/s17112650

## Description

### PRIORITY AND CROSS-REFERENCE TO RELATED APPLICATION(S)

This patent application claims priority based upon the following prior United States patent application(s): (i) "SYSTEM, METHOD AND ARCHITECTURE FOR FACILITATING REMOTE PATIENT CARE", Application No.: 16/449,056 (Docket No.: 13621US01), filed June 21, 2019, in the name(s) of Germinal Ibarrola and Scott DeBates.

### TECHNICAL FIELD

The present disclosure generally relates to remote patient care. More particularly, and not by way of any limitation, the present disclosure is directed to a system, method and cloud-based service architecture for providing remote patient care in a secure network environment.

### BACKGROUND

Implantable medical devices have changed how medical care is provided to patients having a variety of chronic illnesses and disorders. For example, implantable cardiac devices improve cardiac function in patients with heart disease by improving quality of life and reducing mortality rates. Respective types of implantable neurostimulators provide a reduction in pain for chronic pain patients and reduce motor difficulties in patients with Parkinson's disease and other movement disorders. A variety of other medical devices are proposed and are in development to treat other disorders in a wide range of patients.

Many implantable medical devices and other personal medical devices are programmed by a physician or other clinician to optimize the therapy provided by a respective device to an individual patient. Typically, the programming occurs using short-range communication links (e.g., inductive wireless telemetry) in an in-person or in-clinic setting. Since such communications typically require close immediate contact, there is only an extremely small likelihood of a third-party establishing a communication session with the patient's implanted device without the patient's knowledge.

Remote patient care is a healthcare delivery method that aims to use technology to provide patient health outside of a traditional clinical setting (e.g., in a doctor's office or a patient's home). It is widely expected that remote patient care may increase access to care and decrease healthcare delivery costs. Although the adoption of longer range telemetry capabilities and remote care networks provides a number of clinical benefits to patient care, there is an increased risk of malicious parties inappropriately accessing patient data and/or affecting the medical therapy mediated by implanted or other personal medical devices.

EP-B1-2185065 discloses a system for transporting medical information over a wireless network, in which a portable patient communicator is paired with a patient implantable medical device to facilitate communications with a remote server via a wireless network. US-A1-2017/0325091 discloses a system for establishing wireless communication channels between two or more medical devices at an emergency care scene in order to exchange patient data. US-A1-2002/0082665 discloses a medical information communication system that permits monitoring the performance of an implantable medical device implanted within a patient, monitoring the health of the patient, or remotely delivering a therapy to the patient.

### SUMMARY

Aspects of the invention are set out in the independent claims and preferred features are set out in the dependent claims. Embodiments of the present patent disclosure are broadly directed to a system, method and a network architecture for facilitating remote care therapy via secure communication channels between clinicians and patients having one or more implantable medical devices (IMDs), wherein certain unique information retrieved from an IMD is used in association with an encryption key infrastructure system for establishing trusted relationships between a clinician device, a patient's device and the patient's IMD. In one aspect, an embodiment of the present disclosure is directed to a method for facilitating remote care therapy for a patient having an IMD, wherein the claimed method comprises, inter alia, registering a first external device (e.g., a clinician device or programmer) with a remote care session manager disposed in a network and, upon successful registration, obtaining secure communication credentials for the first external device using a key infrastructure system. A trusted association between the first external device and the IMD is established at the remote care session manager when the first external device and the patient are in proximity of each other. Also, a trusted association between the IMD and a second external device (e.g., a patient device or controller) is established at the remote care session manager, preferably after successfully registering and validating the second external device based on at least a portion of information retrieved from the IMD. Thereafter, secure communication credentials are obtained for the second external device using the key infrastructure system. A secure communications channel via a network (e.g., the Internet) may be launched between the first and second external devices after the establishment
of the trusted associations at the remote care session manager in order to effectuate one or more remote care therapy operations relative to the patient, wherein the first external device is remote from the second external device and the second external device is in proximity of the patient.

In one arrangement, remote care therapy operations may be mediated between the first external device and the IMD of the patient via the second external device operative as the patient device. In one arrangement, registering the first external device may include validating the first external device against an enterprise mobile device management system operating in association with a cloud-based remote care session manager.

In another aspect, an embodiment of a method operating at a remote care session manager for facilitating remote therapy is disclosed. The claimed embodiment comprises, *inter alia,* receiving a registration request from a clinician programmer (CP) device and validating the CP device in association with an enterprise mobile device management system. After validating the CP device, a request from the CP device is relayed or proxied to a key infrastructure system for facilitating generation of one or more secure communication credentials associated with the CP device. A first bonding request is received from the CP device to create a trusted association between the CP device and an IMD of a patient, wherein the first bonding request includes one or more trust indicia of the IMD. Responsive to the first bonding request, at least a portion of the trust indicia of the IMD received from the CP device is stored and a first bonding relationship record is generated that identifies the CP device as a trusted entity operative to engage in therapy operations involving the IMD. A registration request in association with a second bonding request may be received from a patient controller (PC) device associated with the patient having the IMD, wherein the second bonding request includes one or more trust indicia retrieved from the IMD. Trust indicia received from the PC device in the second bonding request may be validated against the stored trust indicia received in the first bonding request from the CP device. Thereafter, a request from the PC device may be relayed or proxied to the key infrastructure system for facilitating generation of one or more secure communication credentials associated with the PC device. In one arrangement, trust indicia of the IMD may comprise at least one of a serial number of the IMD, a read-out of an on-board real-time clock (RTC) of the IMD, a timestamp indicating when the IMD is implanted in the patient, one or more device keys and/or device identifiers stored in the IMD, one or more patient biometric data stored in the IMD, and one or more program identifiers and/or validation data associated with therapy programming data stored in the IMD. In one arrangement, the key infrastructure system may comprise a public key infrastructure (PKI) system configured to provide a first key pair including a first public key and a first private key, the first key pair associated with at least one of the CP device and an authorized clinician associated therewith. In a further arrangement, the PKI system may be configured to provide a second key pair including a second public key and a second private key, the second key pair associated with at least one of the PC device and the patient.

In still further aspects, one or more embodiments of a non-transitory computer-readable medium or distributed media containing computer-executable program instructions or code portions stored thereon are disclosed for performing one or more embodiments of the methods of the present invention when executed by a processor entity of a network node, apparatus, system, network element, a datacenter node or cloud platform, and the like, *mutatis mutandis.*

Additional/alternative features and variations of the embodiments as well as the advantages of the present invention will be apparent in view of the following description and accompanying Figures.

### BRIEF DESCRIPTION OF THE DRAWINGS

Embodiments of the present disclosure are illustrated by way of example, and not by way of limitation, in the Figures of the accompanying drawings in which like references indicate similar elements. It should be noted that different references to "an" or "one" embodiment in this disclosure are not necessarily to the same embodiment, and such references may mean at least one. Further, when a particular feature, structure, or characteristic is described in connection with an embodiment, it is submitted that it is within the knowledge of one skilled in the art to effectuate such feature, structure, or characteristic in connection with other embodiments whether or not explicitly described.

The accompanying drawings are incorporated into and form a part of the specification to illustrate one or more exemplary embodiments of the present disclosure. Various advantages and features of the disclosure will be understood from the following Detailed Description taken in connection with the appended claims and with reference to the attached drawing Figures in which:
FIG. 1 depicts an example network environment wherein one or more embodiments of a remote care therapy application or service may be implemented in accordance with the teachings herein;
FIGS. 2A-2C depict flowcharts illustrative of blocks, steps and/or acts that may be (re)combined in one or more arrangements with or without additional flowcharts of the present disclosure for facilitating remote care therapy with respect to a patient having an implantable medical device (IMD) according to one or more embodiments;
FIGS. 3-5 depict flow diagrams illustrative of example message/work flows for effectuating trusted associations among a clinician device, patient device and the patent's IMD via a remote care session manager according to one or more embodiments;
FIGS. 6A-6D depict flowcharts illustrative of additional blocks, steps and/or acts that may be (re)combined in one or more arrangements with or without additional flowcharts of the present disclosure for facilitating remote care therapy according to one or more embodiments;
FIG. 7 depicts a flowchart illustrative of blocks, steps and/or acts that may take place at a remote care session manager for purposes an example embodiment of the present invention;
FIG. 8 depicts a flowchart illustrative of a remote care therapy scenario according to an example embodiment of the present invention;
FIGS. 9A and 9B illustrate representative scenarios for facilitating therapy interactions between a patient's IMD and one or more external devices for purposes of an example embodiment of the present invention;
FIG. 10 depicts a block diagram of a personal or patient medical device according to some example embodiments;
FIG. 11 depicts a block diagram of a clinician device according to some example embodiments;
FIG. 12 depicts a simplified block diagram of a system for initiating a bi-directional communication link for purposes of an example embodiment of the present invention;
FIG. 13 depicts a block diagram of an IMD and associated system that may be configured for facilitating a remote care therapy application for purposes of an example embodiment of the present invention; and
FIG. 14 depicts a block diagram involving a plurality of modules that may be configured as a computer-implemented network node or platform operative as a remote care session manager according to an example embodiment of the present invention.

### DETAILED DESCRIPTION

In the description herein for embodiments of the present disclosure, numerous specific details are provided, such as examples of circuits, devices, components and/or methods, to provide a thorough understanding of embodiments of the present disclosure. One skilled in the relevant art will recognize, however, that an embodiment of the disclosure can be practiced without one or more of the specific details, or with other apparatuses, systems, assemblies, methods, components, materials, parts, and/or the like. In other instances, well-known structures, materials, or operations are not specifically shown or described in detail to avoid obscuring aspects of embodiments of the present disclosure. Accordingly, it will be appreciated by one skilled in the art that the embodiments of the present disclosure may be practiced without such specific components. It should be further recognized that those of ordinary skill in the art, with the aid of the Detailed Description set forth herein and taking reference to the accompanying drawings, will be able to make and use one or more embodiments without undue experimentation.

Additionally, terms such as "coupled" and "connected," along with their derivatives, may be used in the following description, claims, or both. It should be understood that these terms are not necessarily intended as synonyms for each other. "Coupled" may be used to indicate that two or more elements, which may or may not be in direct physical or electrical contact with each other, co-operate or interact with each other. "Connected" may be used to indicate the establishment of communication, i.e., a communicative relationship, between two or more elements that are coupled with each other. Further, in one or more example embodiments set forth herein, generally speaking, an electrical element, component or module may be configured to perform a function if the element may be programmed for performing or otherwise structurally arranged to perform that function.

Some example embodiments described herein may relate to remote care therapy applications particularly set forth with respect to an implantable pulse generator (IPG) or neuromodulator for providing therapy to a desired area of a body or tissue based on a suitable stimulation therapy application, such as a spinal cord stimulation (SCS) system or other neuromodulation systems. However, it should be understood that example embodiments disclosed herein are not limited thereto, but have broad applicability, including but not limited to remote care therapy applications involving different types of implantable devices such as neuromuscular stimulators and sensors, dorsal root ganglion (DRG) stimulators, deep brain stimulators, cochlear stimulators, retinal implanters, muscle stimulators, tissue stimulators, cardiac stimulators or pacemakers, gastric stimulators, and the like, as well as implantable drug delivery/infusion systems, implantable devices configured to effectuate real-time measurement/monitoring of one or more physiological functions of a patient's body (i.e., patient physiometry), including various implantable biomedical sensors and sensing systems. Further, whereas some example embodiments of remote care therapy applications may involve implantable devices, additional and/or alternative embodiments may involve external personal devices, e.g., wearable biomedical devices, that may be configured to provide therapy to the patients analogous to the implantable devices. Accordingly, all such devices may be broadly referred to as "personal medical devices," "personal biomedical instrumentation," or terms of similar import, at least for purposes of some example embodiments of the present disclosure.

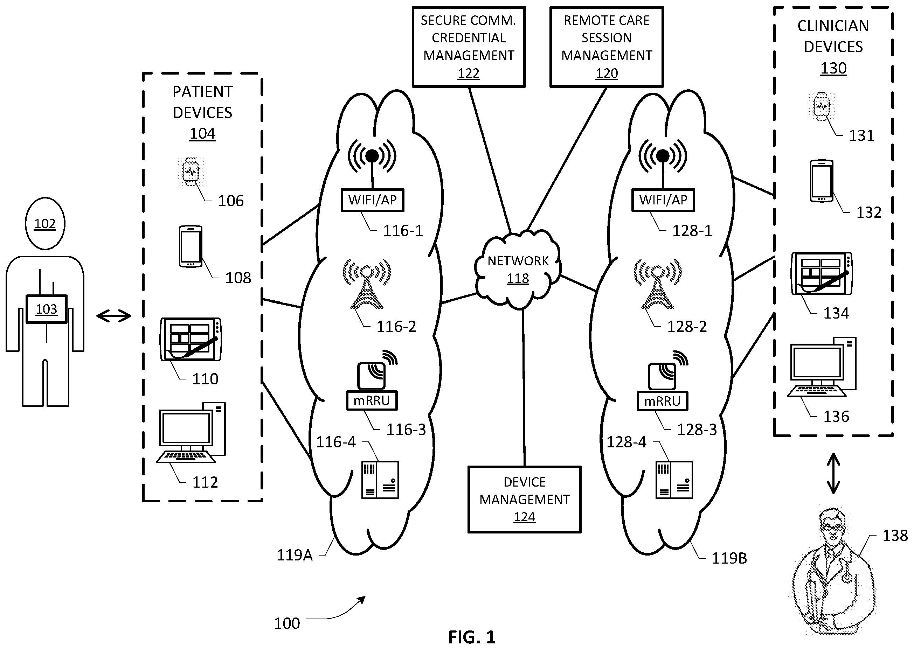

Referring to FIG. 1 in particular, depicted therein is an example network environment 100 wherein one or more embodiments of a remote care therapy application or service may be implemented in accordance with the teachings herein. In general, "remote care therapy" may involve any care, biomedical monitoring, or therapy that may be provided by a clinician, a medical professional or a healthcare provider, and/or their respective authorized agents (including digital/virtual assistants), with respect to a patient over a communications network while the patient and the clinician/provider are not in close proximity of each other (e.g., not engaged in an in-person office visit or consultation). Accordingly, in some embodiments, a remote care therapy application may form a telemedicine or a telehealth application or service that not only allows healthcare professionals to use electronic communications to evaluate, diagnose and treat patients remotely, thereby facilitating efficiency as well as scalability, but also provides patients with relatively quick and convenient access to diversified medical expertise that may be geographically distributed over large areas or regions, preferably via secure communications channels as will be set forth in detail further below.

Illustratively, the example network environment 100 may comprise any combination or sub-combination of a public packet-switched network infrastructure (e.g., the Internet or worldwide web, also sometimes referred to as the "cloud"), private packet-switched network infrastructures such as Intranets and enterprise networks, health service provider network infrastructures, and the like, any of which may span or involve a variety of access networks, backhaul and core networks in an end-to-end network architecture arrangement between one or more patients, e.g., patient(s) 102, and one or more authorized clinicians, healthcare professionals, or agents thereof, e.g., generally represented as caregiver(s) or clinician(s) 138. Example patient(s) 102, each having a suitable implantable device 103, may be provided with a variety of corresponding external devices for controlling, programming, otherwise (re)configuring the functionality of respective implantable device(s) 103, as is known in the art. Such external devices associated with patient(s) 102, referred to herein as patient devices 104, may comprise a variety of user equipment (UE) devices, tethered or untethered, that may be configured to engage in remote care therapy sessions according to some embodiments described below. By way of example, patient devices 104 may comprise smartphones, tablets or phablets, laptops/desktops, handheld/palmtop computers, wearable devices such as smart glasses and smart watches, personal digital assistant (PDA) devices, smart digital assistant devices, etc., any of which may operate in association with one or more virtual assistants, smart home/office appliances, smart TVs, virtual reality (VR), mixed reality (MR) or augmented reality (AR) devices, and the like, which are generally exemplified by wearable device(s) 106, smartphone(s) 108, tablet(s)/phablet(s) 110 and computer(s) 112. As such, example patient devices 104 may include various types of communications circuitry or interfaces to effectuate wired or wireless communications, short-range and long-range radio frequency (RF) communications, magnetic field communications, etc., using any combination of technologies, protocols, and the like, with external networked elements and/or respective implantable devices 103 corresponding to patient(s) 102. With respect to networked communications, patient devices 104 may be configured, independently or in association with one or more digital/virtual assistants, smart home/premises appliances and/or home networks, to effectuate mobile communications using technologies such as Global System for Mobile Communications (GSM) radio access network (GRAN) technology, Enhanced Data Rates for Global System for Mobile Communications (GSM) Evolution (EDGE) network (GERAN) technology, 4G Long Term Evolution (LTE) technology, Fixed Wireless technology, 5^{th} Generation Partnership Project (5GPP or 5G) technology, Integrated Digital Enhanced Network (IDEN) technology, WiMAX technology, various flavors of Code Division Multiple Access (CDMA) technology, heterogeneous access network technology, Universal Mobile Telecommunications System (UMTS) technology, Universal Terrestrial Radio Access Network (UTRAN) technology, All-IP Next Generation Network (NGN) technology, as well as technologies based on various flavors of IEEE 802.11 protocols (e.g., WiFi), and other access point (AP)-based technologies and microcell-based technologies such as femtocells, picocells, etc. Further, some embodiments of patient devices 104 may also include interface circuitry for effectuating network connectivity via satellite communications. Where tethered UE devices are provided as patient devices 104, networked communications may also involve broadband edge network infrastructures based on various flavors of Digital Subscriber Line (DSL) architectures and/or Data Over Cable Service Interface Specification (DOCSIS)-compliant Cable Modem Termination System (CMTS) network architectures (e.g., involving hybrid fiber-coaxial (HFC) physical connectivity). Accordingly, by way of illustration, an edge/access network portion 119A is exemplified with elements such as WiFi/AP node(s) 116-1, macro/microcell node(s) 116-2 and 116-3 (e.g., including micro remote radio units or RRUs, base stations, eNB nodes, etc.) and DSL/CMTS node(s) 116-4.

In similar fashion, example clinicians and/or clinician agents 138 may be provided with a variety of external devices for controlling, programming, otherwise (re)configuring or providing therapy operations with respect to one or more patients 102 mediated via respective implantable device(s) 103, in a local therapy session and/or remote therapy session, depending on implementation and use case scenarios. External devices associated with clinicians/agents 138, referred to herein as clinician devices 130, may comprise a variety of UE devices, tethered or untethered, similar to patient devices 104, which may be configured to engage in remote care therapy sessions as will be set forth in detail further below. Clinician devices 130 may therefore also include devices (which may operate in association with one or more virtual assistants, smart home/office appliances, VRAR virtual reality (VR) or augmented reality (AR) devices, and the like), generally exemplified by wearable device(s) 131, smartphone(s) 132, tablet(s)/phablet(s) 134 and computer(s) 136. Further, example clinician devices 130 may also include various types of network communications circuitry or interfaces similar to that of personal devices 104, which may be configured to operate with a broad range of technologies as set forth above. Accordingly, an edge/access network portion 119B is exemplified as having elements such as WiFi/AP node(s) 128-1, macro/microcell node(s) 128-2 and 128-3 (e.g., including micro remote radio units or RRUs, base stations, eNB nodes, etc.) and DSL/CMTS node(s) 128-4. It should therefore be appreciated that edge/access network portions 119A, 119B may include all or any subset of wireless communication means, technologies and protocols for effectuating data communications with respect to an example embodiment of the present invention.

In one arrangement, a plurality of network elements or nodes may be provided for facilitating a remote care therapy service involving one or more clinicians 138 and one or more patients 102, wherein such elements are hosted or otherwise operated by various stakeholders in a service deployment scenario depending on implementation (e.g., including one or more public clouds, private clouds, or any combination thereof). According to the teachings herein, a remote care session management node 120 is provided, preferably disposed as a cloud-based element coupled to network 118, that is operative in association with a secure communications credentials management node 122 and a device management node 124, to effectuate a trust-based communications overlay/tunneled infrastructure in the network environment 100 whereby a clinician may advantageously engage in a remote care therapy session with a particular client, as will be described below in reference to the remaining drawing Figures.

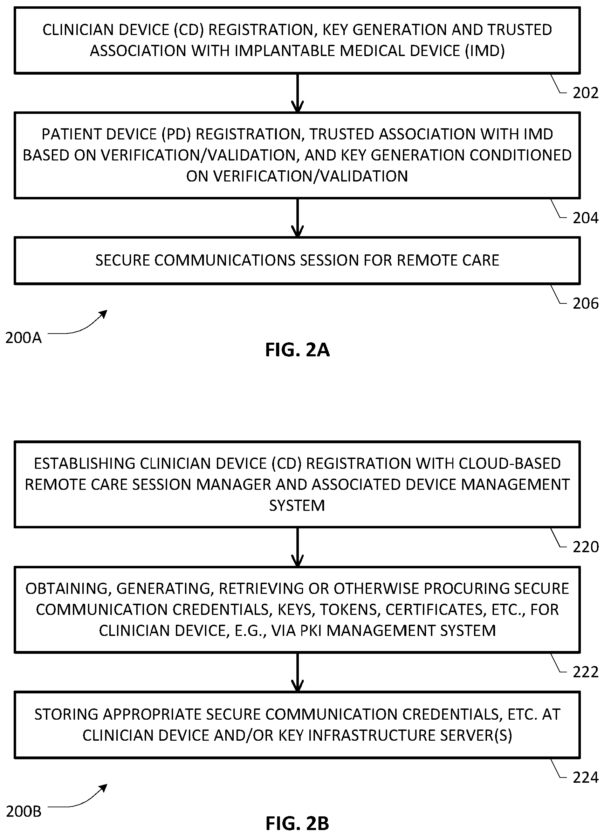

FIGS. 2A-2C depict flowcharts illustrative of blocks, steps and/or acts that may be (re)combined in one or more arrangements with or without additional flowcharts of the present disclosure for facilitating remote care therapy with respect to a patient having an IMD. Broadly, embodiments herein utilize certain unique information from a patient's IMD in association with a suitable key structure system (e.g., a public key infrastructure (PKI) system) operating as a secure credentials management system with respect to a clinician device and a patent device for registration and generation of trust associations, preferably prior to establishing a remote therapy/programming session therebetween. Example process 200A of FIG. 2A is representative of an overall scheme that may be practiced in effectuating a remote care therapy system via a network environment (e.g., network environment 100 shown in FIG. 1) according to an embodiment. At block 202, various operations relating to clinician device registration, encryption key generation and establishment of a trusted association between the clinician device and a patient's IMD are effectuated. At block 204, various operations relating to patient device registration and establishment of trusted association with the IMD based on verification/validation are performed, which are followed by encryption key generation conditioned on verification/validation operations. It will be appreciated that at least a portion of the operations set forth at blocks 202 and 204 may be performed while the clinician and the patient are in proximity of each other (e.g., at the clinician's office, the patient's home, etc., where there is little chance of unauthorized tampering with device communications between the clinician device and the IMD as well as between the patient device and the IMD). After the operations of blocks 202 and 204 are successfully completed, the patient and the clinician may go their separate ways (e.g., the patient may leave the clinician's office or return home, the clinician may leave the office or the patient's premises, etc.), whereby the clinician may be remotely located with respect to the patient. By virtue of the trusted associations established among the devices through the IMD, a secure communication session may be established at a later time, e.g., by the clinician operating the authorized clinician device and/or the patient operating the patient device, for providing/consuming remote care (block 206).

Example process 200B of FIG. 2B is representative of additional details with respect to some of the operations set forth above according to some embodiments. At block 220, a clinician device registers with a cloud-based remote care session manager node and associated device management system. Thereafter, one or more secure communication credentials, keys, tokens, certificates, etc., for the clinician device, may be obtained, generated, retrieved, generated or otherwise procured, e.g., using a PKI management system (block 222). At block 224, the secure communication credentials may be stored at the clinician device. Where public-key cryptographic keys are involved, such keys may also stored/managed via suitable certification/registration authorities.

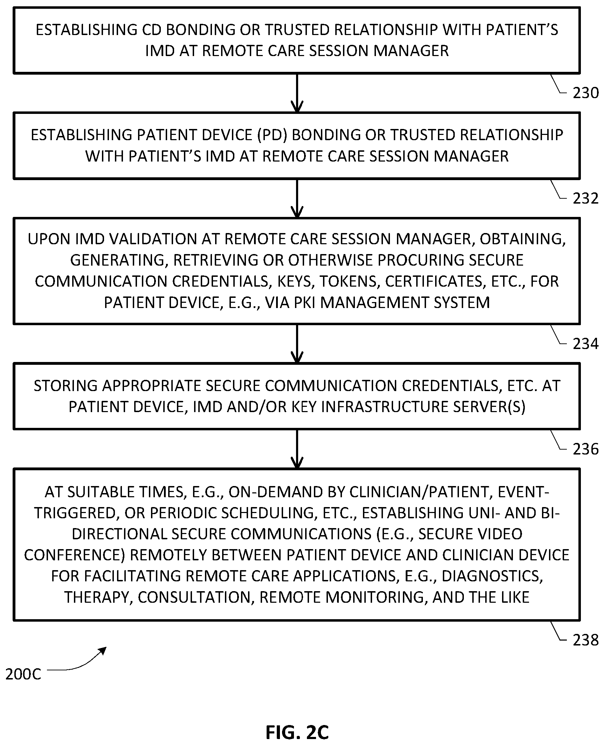

Example process 200C of FIG. 2C is representative of additional details with respect to some of the operations set forth above in reference to FIG. 2A according to some embodiments. At block 230, a device bonding or trusted relationship between the clinician device and the patient's IMD is established at the remote care session manager based on certain unique information obtained/retrieved from the IMD. At block 232, a device bonding or trusted relationship between the patient's device and the patient's IMD is established at the remote care session manager. Upon performing suitable IMD validation operations at the remote care session manager for the patient device, one or more secure communication credentials, keys, tokens, certificates, etc., may be obtained, generated, retrieved, generated or otherwise procured, for the patient device (block 234). Similar to the key generation process for the clinician device set forth in FIG. 2B, the keys for the patient device may be obtained in association with a PKI management system and such keys may be stored/managed at the patient device, key certification/registration authorities, or both. In some example embodiments, at least one or more private keys may also be stored in the patient's IMD, as set forth at block 236. Thereafter, uni- and/or bi-directional secure communications may be established remotely between the patient device and the clinician device (e.g., secure video conferences) for facilitating remote care applications, e.g., diagnostics, therapy, consultation, remote monitoring, and the like. Skilled artisans will recognize that such secure communications may be launched by the clinician and/or the patient, e.g., as needed, on-demand, or effectuated responsive to (pre)configured mechanisms (e.g., triggered by reaching certain thresholds in a stimulation therapy), or based on periodic scheduling, and the like, as generally set forth at block 238.

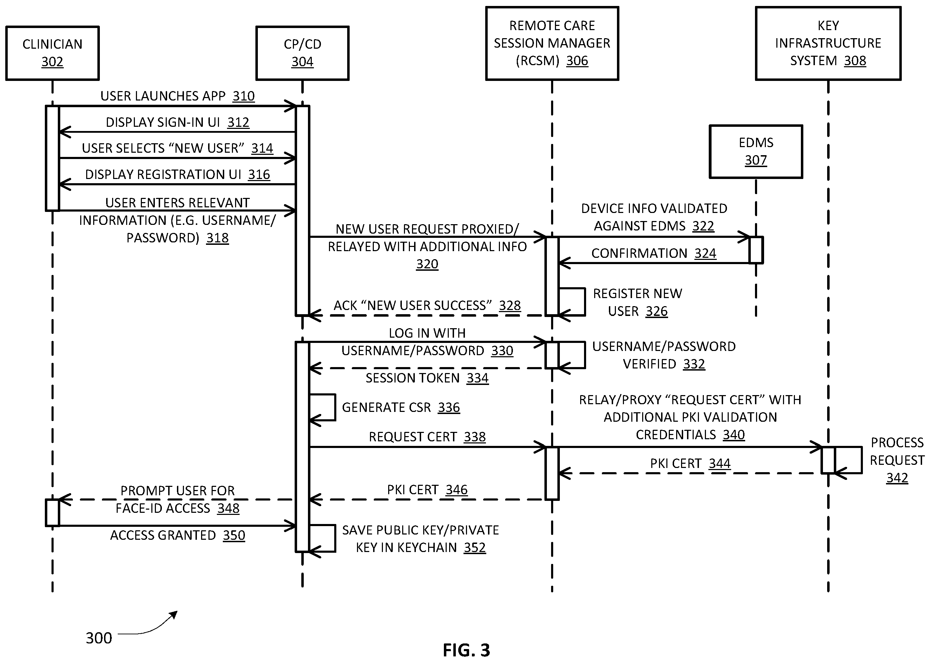

FIGS. 3-5 depict message flow diagrams illustrative of one or more message and/or work flows that exemplify additional details with respect to registering clinician and patient devices and effectuating trusted associations based on the patent's IMD via a remote care session manager according to some embodiments of the present patent disclosure. By way of illustration, message/work flow 300 shown in FIG. 3 is representative of operations that may be undertaken by a clinician 302 who may be a new or current healthcare provider of one or more patients (not explicitly shown in this FIG.), where the clinician 302 may be allowed or otherwise engaged by a suitable healthcare entity, facility, organization or authority to provide remote care therapy with respect to such patient(s) under applicable service arrangements and/or regulations. Accordingly, at least a portion of the operations involving clinician 302 in this message flow diagram may comprise operations relative to initial enrollment/registration of clinician 302 and associated clinician programmer/device (CP/CD) 304. Skilled in the art will recognize that CP/CD 304 may comprise any type of computing/communications device (e.g., selected from clinician devices 130 depicted in FIG. 1), which may be configured to execute a special piece of software, code or program(s) (generally referred to as an "application" or "app") to allow the clinician 302 to perform various preconfigured and/or (re)configurable healthcare-specific tasks via a suitable user interface (UI) that may include graphics, video, audio, and/or text or command line interfacing. In general, such apps may be referred to as desktop applications or mobile apps depending on whether a desktop/laptop device or a handheld mobile device (e.g., a smartphone, a tablet/phablet, or a custom-built clinician programmer device) is configured to operate as CP/CD 304. Regardless of how CD/CP 304 is implemented, skilled artisan will recognize that one or more suitable apps are downloaded or otherwise provided with CP/CD 304 (e.g., either beforehand or at initial enrollment in some embodiments) for executing as part of an application software platform in association with the device Operating System (OS) environment, which execution may be effectuated when the application is launched by the clinician 302 or clinician's authorized agent, e.g., via appropriate voice/graphics/text input, as exemplified at work flow 310 in FIG. 3. Responsive thereto, a UI display is presented to the clinician/agent 302 for facilitating input of appropriate user registration credentials (e.g., usernames, passwords, multi-factor authentication tokens etc.), by way of selecting a "New User" icon, software button or pull-down menu window in the UI display, as exemplified by work flow messages 312, 314, 316, 318 in FIG. 3. Upon entry of relevant user information with respect to the clinician/agent 302, CP/CD 304 relays or proxies a new user registration request to a remote care session manager (RCSM) 306 via a suitable network connection, as exemplified by message flow 320, wherein RCSM 306 may be disposed in a network environment as described above in reference to FIG. 1. In some example embodiments, additional device information associated with CP/CD 304 and/or the remote care application may be included in the relayed/proxied request, such as, e.g., device identity, application version data, and/or any hard-coded device data, etc. RCSM 306 may be provided as part of a cloud-based service architecture, e.g., Software as a Service (SaaS), Platform as a Service (PaaS), Infrastructure as a Service (IaaS) etc., operated/hosted by one or more healthcare providers, medical services providers, medical equipment manufacturers, and the like, as noted previously. In some embodiments, RCSM 306 may be configured to validate the received device information against an enterprise device management system (EDMS) 307 associated therewith, e.g., pursuant to a suitable service level agreement or SLA, in order to ensure that only appropriately managed clinician devices are allowed to engage in remote care service enrollment. In one embodiment, EDMS 307 may be implemented a mobile device lifecycle management system supported by a third-party service provider operative to deploy, configure, manage, support and secure mobile clinician devices through mobile device management (MDM) profiles installed on the devices, such as, e.g., CP/CD 304. In some embodiments, MDM software may be configured to facilitate asset inventory, over-the-air (OTA) configuration of application(s), remote troubleshooting, and remote lock and wipe capabilities to secure the devices and the sensitive patient data on them. In one implementation, MDM software operative in association with RCSM 306 may be provided as an application operating on the managed clinician devices that may be configured as part of a device- and platform-agnostic service, which centralizes the management, configuration and security of one or more clinicians and associated devices approved for providing remote care. In some embodiments, the functionalities and capabilities of RCSM and EDMS may be integrated within a datacenter platform, e.g., as a server farm having advantageous features such as failover, redundancy, hot-pluggability and high availability, among others.

Irrespective of whether RCSM 306 and EDMS 307 are integrated (e.g., tightly coupled or loosely coupled), appropriate validation message flow 322 and confirmation message flow 324 may be exchanged therebetween to validate and confirm the new user (i.e., clinician 302 and/or CP/CD 304) for purposes of an embodiment of the present patent disclosure. Responsive thereto, RCSM 306 is operative to register the clinician 302 and associated device CP/CD as a valid, authenticated and/or authorized user/provider, as exemplified by message flow 326. Thereafter, a suitable response or acknowledgement message may be generated by the remote care session manager 306 to CP/CD 304, as exemplified by message flow 328. Subsequently, a log-in operation may be effectuated in association with CP/CD 306 (e.g., substantially immediately after the registration acknowledgement message is received from RCSM 306 or after a time period has passed, and/or after certain preconfigured events and notifications have taken place) to log in with RCSM 306 using appropriate user credentials (e.g., the username and password combination, etc.) for commencing a session with respect to obtaining and/or generating secure communication credentials. Upon verification by RCSM 306, a session token may be provided to CP/CD 304. By way of illustration, these operations are exemplified by message flows 330, 332 and 334 in the message flow diagram 300 of FIG. 3. Responsive thereto, CP/CD 304 may be configured to generate or launch a certificate signing request (CSR) process, exemplified by message flow 336, with respect to one or more cryptographic keys, wherein a request for a digital certificate may be transmitted to RCSM 306, which may be propagated, relayed or otherwise proxied to a suitable key infrastructure system 308, as exemplified by message flows 338 and 340. In some embodiments, where an asymmetric cryptography system is implemented for securing communications (e.g., a key pair comprising a separate public key and a private key is used), the key infrastructure system 308 may comprise a PKI system, as noted previously. In a further embodiment, the proxied request 340 relayed to the key infrastructure system 308 may include additional PKI validation credentials depending on the configuration of RCSM 306 and/or profile management of clinician 302 and associated CP/CD 304. Responsive to processing the certificate request 340, the key infrastructure system 308 is operative to generate a digital certificate, which is propagated to CP/CD 304 via RCSM 306, as exemplified by message flows 342, 344 and 346. In some example embodiments, a further level of user authentication for access control may be required before the secure communication credentials (e.g., public/private keys, tokens, etc.) are stored in CP/CD 304. For example, facial recognition and/or other forms of biometric identification may be required of the clinician 302 prior to access is granted and the keys are securely stored at CP/CD 304, as exemplified by flows 348, 350 and 352.

With respect to managing and maintaining the secrecy of private keys and facilitating assurance of public keys, some example embodiments may therefore involve providing suitable hardware, software and/or firmware as part of CP/CD 304 functionality, wherein a key pair combination may be generated and/or otherwise obtained, e.g., either in association with a certificate or certification authority (CA), or otherwise, such that the private key is stored at an private key store and the public key is provided in a CSR process identifying the requesting subject (e.g., clinician 302, CP/CD 304, or in combination) that may be processed by the CA and/or in association with a third-party registration authority (RA). Further, skilled artisans will recognize that several levels of certificates may be provided depending on the level of security or trust is required in a remote care therapy service, which in turn may necessitate providing different levels of identity credentials as part the CSR. Upon duly processing the CSR and verifying the identity of the requester (e.g., clinician 302, and/or CP/CD 304), a digital certificate may be issued to the requester, which may include requester identity credentials, expiration date, usage levels, certificate issuer information, etc.

While the public key of a subject entity may be stored on the digital certificate, the associated secret private key can be stored on the key owner's device (e.g., CP/CD 304) in some embodiments. However, such an arrangement may not be optimal because if a malicious party gains access to the device, s/he can unlawfully access the private key, thereby breaching or otherwise compromising the level of security and/or privacy required for remote care therapy services. In some embodiments, therefore, the private key may be stored on a secure removable storage or token that can only be accessed via further validation (e.g., a challenge-response mechanism). For example, CP/CD 304 may be provided with a suitable interface to facilitate a security token operative with a variety of interfacing technologies, e.g., Universal Serial Bus (USB), near-field communication (NFC), radio frequency identification (RFID), or Bluetooth, as well as audio/visual signature interfacing. It will be appreciated that such security measures may help reduce the likelihood that a clinician's device is compromised before attempting to "bond" with a patient's IMD (i.e., establishing a trusted association or relationship between the clinician's device and a patient's IMD via RCSM 306). In some example embodiments, establishing such inter-device relationships may be facilitated in a secure or trusted environment to help enhance the overall security of a remote care therapy service scenario.

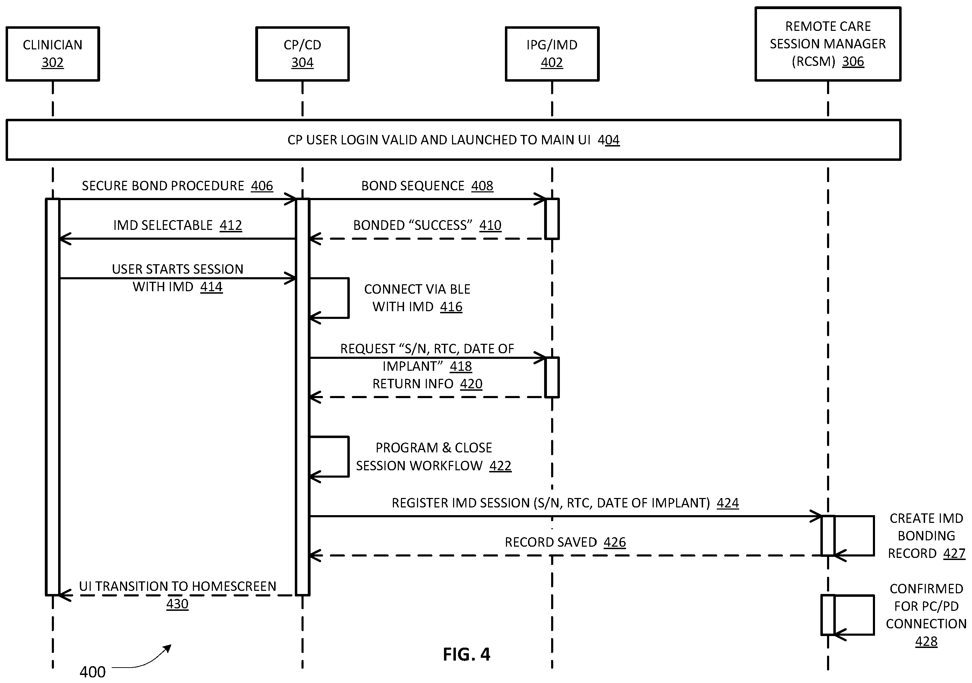

Turning now to FIG. 4, message/work flow 400 depicted therein is representative of operations that may be undertaken by the clinician 302 preferably in the presence of a patient (not explicitly shown) having an IMD 402 for securely bonding with and registering IMD 402 RCSM 306 in order to facilitate remote care therapy at some point in the future. As one skilled in the art will recognize, the message/work flow 400 of FIG. 4 and the message/work flow 300 of FIG. 3 may be engaged by the clinician 302 at different times, although it is preferred that the message/work flow 300 takes place prior to the message/work flow 400. Further, whereas the message/work flow 300 of FIG. 3 may typically take place without the presence of a patient (e.g., remotely, because the patient's IMD is not involved), such message/work flow may also be engaged while in the presence or vicinity of the patient (e.g., locally, when engaging with the patient's IMD to perform at least a portion of the operations of the message/work flow 400). Illustratively, the message/work flow 400 may be engaged in one embodiment during an in-person or in-clinic visit (e.g., an initial patient visit with a new clinician or with an existing clinician when the patient has acquired a new/replacement IMD, etc.) while the patient having the IMD 402 is in the clinician's office or offsite at a medical care facility or even at the patient's home or some type of care facility or affiliate (e.g., a nursing home or eldercare facility). Generally, in one arrangement, the clinician 302 operating CP/CD 304 may be already validly logged in and engaged in suitable network connectivity with RCSM 308, as exemplified by log-in session 404. A secure "bonding" procedure 406 may be initiated by the clinician 402 via CP/CD 304, e.g., using suitable software menu options, pull-down menus, icons, or other mechanisms, etc., to cause a bonding sequence 408 to be launched between CP/CD 304 and the patient's IMD 402. In one arrangement, such secure bonding procedure may be facilitated by using a "triggering device" in the proximity or vicinity of IMD 402, as will be set forth in additional detail hereinbelow. Responsive to receiving a message or indication that the bonding sequence is successful, as exemplified by message flow 410, an indication 412 (e.g., visual, textual or audio/video) may be provided to the clinician 302 that IMD 402 is selectable for initiating a secure local communication session therewith via CP/CD 304. In one embodiment, such a local communication session between may be effectuated using without limitation, *inter alia,* Bluetooth Low Energy (BLE), NFC, Zigbee, and the like, as exemplified by message flows 414, 416. After establishing the communication channel between CP/CD 304 and IMD 402, a request for information may be generated to IMD 402, which in some embodiments may comprise a request for the IMD's serial number, time/date of when the IMD 402 is implanted in the patient (e.g., a timestamp indicative of first implantation and/or subsequent (re)implantations if performed), a measurement or read-out of any device, circuit or module configured to keep an accurate record of the current time and/or lapsed time, e.g., high precision event timers (e.g., based on hardware, software and/or firmware events), real-time clocks (RTCs), timestamp counters (TSCs), power management timers, programmable interval timers (PITs), etc., as well as other unique data, information, or indicia stored in the IMD 402 (e.g., one or more device-based cryptographic keys, IMD's device identifiers, etc.). In addition, depending on implementation, one or more biometric authentication data and/or a digest thereof uniquely associated with the patient (e.g., digital fingerprints, iris/retinal scans, facial pattern recognition, voice recognition, etc.) as well as certain programmatically generated data (e.g., based on hash algorithms, etc.) performed on stored patient data, program/version identifiers associated with stored stimulation/therapy programs, and the like, may also be requested and/or obtained as part of the requesting process 418. Any combination or sub-combination of the foregoing data, without limitation, as well as any other personalized authentication indicia associated with a patient (e.g., based on genetic/chromosomal markers such as specific sequences of Deoxyribonucleic acid or DNA, immunological/physiological markers, digital challenge-response patterns such as brain scan waveforms responsive to an audio/visual challenge, and the like), currently known or heretofore unknown, may be referred to herein as "trust indicia," which may be returned to the requesting CP/CD 304 in a secure local communication session. As will be set forth further below, any portion of such trust indicia may be utilized in registering an IMD and creating a trusted associative relationship involving the unique combination of a clinician programmer/device and a patient controller/device, and mediated via the corresponding IMD for purposes of an embodiment of the present patent disclosure.

Responsive to receiving the requested data from IMD 402, the clinician 302 may operate CP/CD 304 to program IMD 402 (e.g., input appropriate therapy settings, etc.) and, in one embodiment, may close the secure local communication session as exemplified as message/work flows 420, 422. Thereafter, the clinician 302 may launch an IMD registration session via CP/CD 304, e.g., using pull-down menus, icons, or other mechanisms, etc., available as part of the downloaded remote care therapy app, with RCSM 306 by means of a network connection (e.g., a broadband connection effectuated via WiFi, 5G, LTE, CDMA/TDMA, WiMAX, DSL/CMTS, etc.) therewith, wherein a registration message including at least a portion of the trust indicia data may be transmitted to RCSM 306 as exemplified by message flow 424. Responsive thereto, RCSM 306 is operative to create and store a bonding relationship record 427 thereat, which in some embodiments may include the trust indicia data forwarded by CP/CD 304 as well as suitable identity/indication data corresponding to the clinician 302 and/or CP/CD 304, e.g., to indicate that clinician 302 and/or CP/CD 304 are trusted entities operative to engage in one or more therapy operations involving the patient's IMD 402. A response message 426 may be generated back to CP/CD 426 from RCSM 306 indicating the generation and storage of the IMD bonding relationship record. In an example embodiment, the UI display of CP/CD 304 may transition to a home screen to allow the clinician 302 to undertake further operations and/or close and exit the IMD registration process (e.g., engaging in local therapy administration, (re)configuration of program settings, etc.). Separately, RCSM 306 may be configured to maintain/remain in a state conditioned for accepting and/or engaging in further operations relative to creating a binding relationship between IMD 402 and a patient device or controller, as exemplified by work flow 428, which may take place, for example, at a later time at the discretion of the patient in a separate registration process. Skilled artisans will recognize, however, that in some example scenarios it may be more optimal for a patient to engage in such a registration procedure with RCSM 306 following the clinician's IMD registration process while still involved in an in-person consultation with the patient because the clinician 302 and/or agent may be able to assist the patient with the process in a more personal and/or helpful setting.

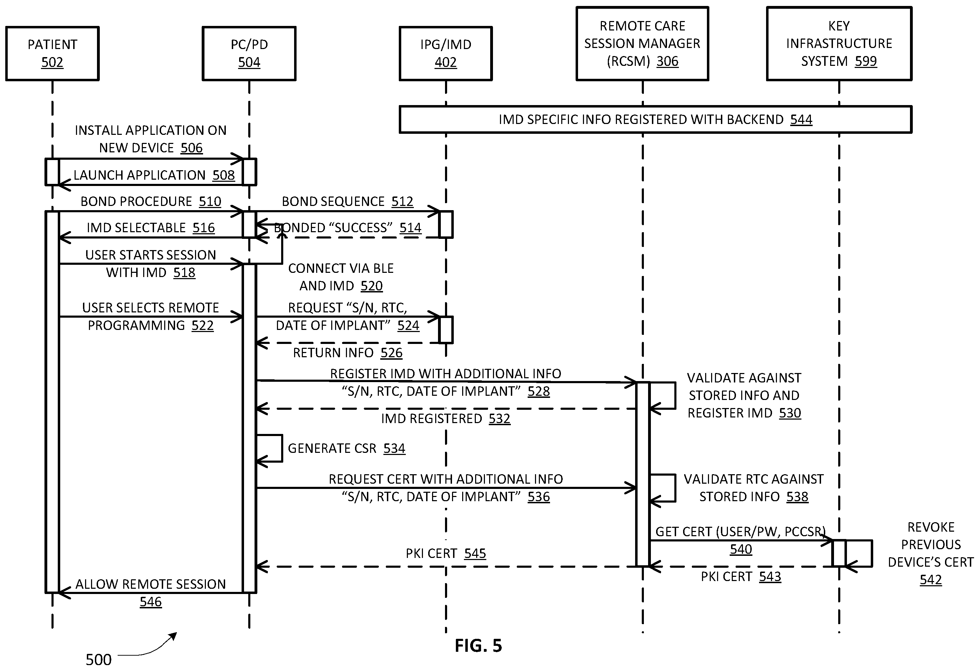

FIG. 5 depicts a message/work flow diagram 500 representing operations that may be undertaken by a patient 502 having an implantable pulse generator or other device, e.g., IMD 402, for registering a patient controller or device (PC/PD) 504 at RCSM 306 and associating it with IMD 402 via a trusted relationship established thereat. In general, the patient 502 may undertake the registration process with or without a clinician being present, as noted above. Further, whereas the message/work flow 400 of FIG. 4 and the message/work flow 500 of FIG. 5 may be engaged by the clinician 302 and the patient 502 at different times (or even in different places), it is preferred that the message/work flow 400 takes place prior to the message/work flow 500. In other words, an IMD registration process involving the clinician 302 registering the patient's IMD 402 and causing the storage of the IMD's trust indicia at RCSM 306 preferably takes place prior to the patient 502 engaging in the message/work flow 500 for purposes of an example embodiment of the present disclosure. Accordingly, such backend registration of IMD-specific data (i.e., trust indicia) is exemplified as set forth at work flow block 544 in FIG. 5. Initially, the patient 502 operating PC/PD 504 may install, download or otherwise acquire a medical therapy application or "app" (e.g., via a server push, client pull, etc.) from an appropriate organization, healthcare entity, facility, affiliate or service provider, which may be launched or caused to be executed to present a suitable UI having various selectable options to the patient 502 in relation to device initialization, registration, therapy options and modes, and the like, as exemplified by message/work flows 506 and 508 between the patient 502 and PC/PD 504. By selecting IMD bonding mode option, a secure bonding procedure or sequence may be engaged between PC/PD 504 and the patient's IMD 402, as exemplified by message/work flows 510 and 512. In one example embodiment, the secure bonding procedure engaged by the patient 502 via PC/PD 504 may be substantially similar to the clinician's secure bonding procedure 406 set forth above, which will be further described in additional detail hereinbelow. Upon receiving a confirmation 514 that the bonding with IMD 402 is successful, an indication 516 may be provided to the patient 502 that the patient's IMD 402 is selectable for commencing a local communication session therewith using appropriate local connectivity. As with the clinician's local communications with IMD 402 (i.e., engaged when the clinician 302 and the patient 502 in proximity of each other in an in-person consultation visit), the patient's local communications with IMD 402 may be effectuated via a variety of short-range communications technologies provided with PC/PD 504, e.g., BLE, NFC, etc., as exemplified by message/work flows 518 and 520, essentially in a secure manner because of the extremely low likelihood that a malicious attacker would be able to intrude into the patient's personal space without the patient's consent or knowledge.

Upon establishing a suitable local communications channel with IMD 402, the patient 502 may select a remote programming mode to cause the generation and transmission of a device data request to IMD 524 by PC/PD 504 as exemplified by message/work flows 522 and 524. In one example embodiment, the device data request 524 may comprise a request for at least a portion of the IMD trust indicia, which may include various types of device-specific information as noted above. Responsive to receiving the requested trust indicia in a response message 526 from IMD 402, PC/PD 504 may be caused to generate and transmit a device registration request 528 including at least a portion of the trust indicia to RCSM 306 via a suitable network connection using any type of telecommunications technologies as previously noted. Responsive to the device registration request, RCSM 306 is operative to validate the received trust indicia against the stored trust indicia recorded as part of the message/work flow 400 of FIG. 4 described above. Upon successful validation of the trust indicia transmitted from PC/PD 504, IMD 402 is registered and an association record may be established indicating a trusted relationship between IMD 402 and the patient 502 and/or corresponding PC/PD 504, as exemplified by work flow 530. Thereafter, a suitable response message 532 may be generated and transmitted to PC/PD 504 that IMD 402 is registered at RCSM 306.

Subsequent to successful registration of IMD 402, PC/PD 504 may engage in a CSR process 534 with respect to one or more cryptographic keys (e.g., similar to the CSR process 336 set forth in the message/work flow 300 of FIG. 3). Accordingly, a request 536 for a digital certificate may be transmitted to RCSM 306, wherein the request 536 may include at least a portion of the IMD trust indicia. Responsive to the certificate request 536, RCSM 306 is operative to verify at least a portion of the received IMD trust indicia against the corresponding portions of the stored IMD trust indicia for a further validation 538. In one embodiment, a received RTC value may be compared against the stored RTC value to verify that both values match after accounting for the passage of a certain known time period. As there are two separate validations being performed at RCSM 306, albeit at different times, as exemplified by work flows 530 and 538, they may be referred to as first and second validations, respectively, for at least some embodiments of the present invention. Upon confirming that the second validation 538 is successful, RCSM 306 may propagate or relay a certificate request 540 containing additional credentials, e.g., patient's username, password, etc., depending on the level of trust required in a remote care therapy service, as well as the CSR information to a suitable key infrastructure system 599, as exemplified by message flow 540. In some embodiments, the key infrastructure system 599 employed for obtaining/generating cryptographic keys and certificates with respect to the patient 502 and/or associated PC/PD 504 may be the same as the key infrastructure system 308 employed with respect to the clinician 302 and/or associated CP/CD 304 although it is not a necessary condition. Regardless of whether two separate key infrastructure systems are utilized, skilled artisans will appreciate that where PKI systems are used for facilitating key creation, key establishment, key storage, key archival and destruction, substantially similar cryptographic processes may take place with respect to both clinician-specific and patient-specific message/work flows. Accordingly, the description set forth hereinabove with respect to the relevant portions of FIG. 3 is also equally applicable with respect the cryptographic and certificate request process of FIG. 5, *mutatis mutandis.* For example, some example embodiments may therefore involve providing suitable hardware, software and/or firmware as part of PC/PD 504 functionality for generating and/or obtaining a public key - private key pair combination in association with a suitable CA/RA, wherein the private key may be stored in a secure private key storage area of PC/PD 504, IMD 402 or some other trusted entity.

In one example embodiment, if the patient 502 and/or associated PC/PD 504 was issued a previous certificate, it may be revoked by a suitable CA/RA of the key infrastructure system 599 prior to issuing a valid public key certificate, which may be propagated or otherwise relayed back PC/PD 504 as exemplified by message/work flows 542, 543 and 545. Responsive to obtaining secure communication credentials, PC/PD 504 may be configured to provide an indication 546 that the patient 502 is allowed to launch a remote care therapy session with the clinician 302, e.g., which can be either substantially immediately or at the patient's discretion, or at a subsequent time in a dynamically configured event-triggered manner.

In view of the foregoing description, it should be appreciated that devices associated with clinicians and patients for (re)programming, (re)configuring or otherwise manipulating therapy programs, settings and/or sensing functionalities of an IMD, such as CP/CD 304 and PC/PD 504, for example, may be characterized as external devices, which may be designated as a first external device, a second external device, and so on, depending on the context and/or implementation scenario. Further, as noted elsewhere in the present patent disclosure, a patient may be provided with a plurality of external devices operative with one or more IMDs of the patient (e.g., having a first IMD for SCS therapy, a second IMD for DRG therapy, a third IMD for DBS therapy, etc.), whereas the clinicians may likewise be provided with a plurality of external devices, each of which may be configured to interoperate with one or more patients and/or one or more IMDs for providing remote care therapy operations with respect to one or more therapy applications. Regardless of what specific mix of clinician devices, patient devices and IMDs is deployed in a particular remote care service scenario at network level, an embodiment of the present invention may be configured to provide a trusted association for each device triplet of a first external device (e.g., a clinician device), a second external device (e.g., a patient device) and an IMD of the patient using the IMD trust indicia as set forth herein in order to facilitate a respective remote care service session, wherein a cloud-based RCSM may be advantageously configured to serve the entire network of the deployed devices.

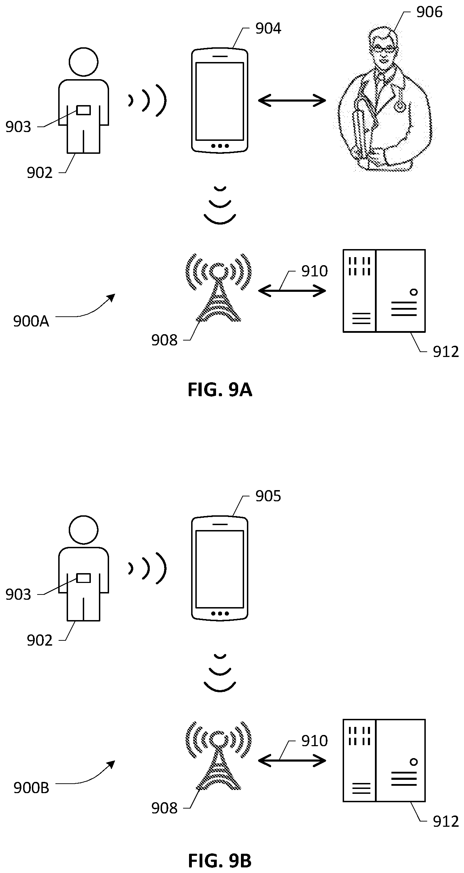

FIGS. 9A and 9B illustrate representative scenarios for facilitating therapy operations and associated interactions between a patient's IMD and one or more external devices for purposes of an example embodiment of the present invention. In particular, FIG. 9A depicts a system-level scenario 900A in which an IMD may be programmed by a clinician device operating as a first external device according to some embodiments. FIG. 9B similarly depicts a system-level scenario 900B in which an IMD may be configured to communicate with a patient controller/device operating as a second external device, which in turn may communicate with one or more remote device management servers according to some embodiments.

With respect to the scenario 900A of FIG. 9A, an IMD 903 is illustratively shown as implanted within a patient 902 at a suitable place proximate to a tissue/organ region of the patient 902 intended for therapy/monitoring. At appropriate times, IMD 903 is operative to communicate with a first external device, e.g., clinician programmer/device 904, which is operated by a clinician or authorized agent, collectively shown as clinician 906. The programming clinician 906 utilizes one or more UI screens/windows of external device 904 to define or control a therapy provided to patient 902 mediated by the IMD 903. The clinician(s) 906 may define or set one or more therapy parameters provided as part of a therapy application. For example, without limitation, the clinician 906 may define pulse amplitudes, pulse frequencies, pulse patterns, pacing delays, and/or a variety of other therapy parameters depending upon the IMD 903 and the intended therapy for patient 902.

During a programming session, programming data may be communicated from clinician programmer device 904 to one or more remote device management servers 912 via a suitable network infrastructure 908, 910. Preferably, the set of programming data may be subjected to authorization and validation processes to ensure that only programming data from authorized clinicians will accepted by the IMD 903 of patient 902. Suitable security algorithms may be employed to validate and authorize communication between clinician programmer device 904 and servers 912, such as communication of user/clinician identifiers, passwords, device identifiers, network identifiers, security/cryptographic keys, digital certificates, location data, and/or the like.

Servers 912 may also assist in validation and creation of the programming data. For example, servers 912 may compare the programming data submitted by a clinician/agent, e.g., clinician 906, for review by one or more automated validation processes created to optimize therapies based on previously determined clinical data. If there is a discrepancy or a possible improvement, servers 912 may communicate suggested changes to the clinician(s) 906 operating device 904. Also, servers 912 may offer application services to assist the programming process. For example, servers 912 may serve one or more UE screens using a suitable protocol (e.g., HTML) to device 904 to permit the clinician(s) 906 to define/modify the therapy for patient 902.

When the given set of programming data is suitably defined, server(s) 912 may generate data to permit the programming data to control the therapeutic operations of the implanted medical device of patient 902. By way of illustration, if server(s) 912 determine that clinician programmer device 904 is being operated by a properly identified clinician with proper programming permissions, server(s) 912 may generate authorization and/or validation data to accompany the programming data. Server(s) 912 communicate the authorization/validation data to clinician programming device 904 via network infrastructure 908, 910. Clinician programming device 904 communicates the programming data and the authorization/validation data to IMD 903 of patient 902, which may be configured to analyze the authorization/validation data. If the authorization/validation data is determined by IMD 903 to be valid, the IMD may conduct therapy operations (e.g., generating electrical pulses for application to tissue of the patient, delivery of pharmaceuticals, and/or the like) according to the programming data.

As used herein, validation data is data that provides information to ascertain the integrity of the programming data and/or whether the programming data was generated by a properly authorized clinician or other user. Validation data may be generated by generating a value from therapeutic settings and/or programming metadata using a checksum, digest, or other suitable function. The function may include application of one or more cryptographic keys or the result of the function may be varied by application of one or more cryptographic keys. The respective keys used for cryptographic processing may include keys selected according to public-key cryptography or asymmetric cryptography (e.g., RSA (Rivest-Shamir-Adleman) cryptography and Elliptic Curve Cryptography (ECC)), and the like, although other cryptographic keys and techniques may also be used in additional or alternative embodiments.

With respect the example scenario 900B of FIG. 9B, a patient controller/device 905 associated with patient 902 is operative as a second external device, which in turn may communicate with one or more remote device management servers 912. By way of illustration, patient 902 may utilize device 905 for one or more of a variety of tasks relating to therapy operations, IMD maintenance, etc. For example, patient 902 may interact with device 905 to check the status of the patient's IMD 903 (battery level, existing operating mode, etc.). Also, IMD 903 may be configured to monitor physiological signal or processes of the patient 902, which may be stored locally within IMD 903 and/or in the external device 905. For example, patient controller device 905 may communicate with IMD 903 periodically or upon detecting certain triggered events to access stored physiological data, which may be communicated in a remote therapy/monitoring session to a networked entity and/or an authorized clinician. The patient controller device 905 may display a suitable indication of the patient's condition (e.g., heart rate, glucose level, neurological activity, etc.) using audio, visual, textual means. The accessed physiological or other patient data may also be communicated to one or more external servers, e.g., servers 912, which may be accessed by the clinician while engaged in a remote therapy session with the patient 902 in accordance with some embodiments. The physiological data may be analyzed to monitor the patient's condition, for example, to identify if the patient is experiencing undesired physiological conditions, e.g., cardiac conditions such as episodes of tachycardia, arrhythmias, and other conditions. Automated processing may occur to identify relevant medical conditions, e.g., based on artificial intelligence or machine learning techniques in association with "Big Data" analytics. Alerts to the patient 902 and/or to the patient's medical professionals may be provided by the patient controller device 905 and/or server(s) 912 if warranted by the physiological data.

Also, depending upon the type of IMD 903, patient 902 may interact with external device 905 to control some aspects of the patient's therapy. For example, some neuromodulation devices frequently include multiple stimulation programs, and depending upon the patient's experience of pain at any given time, the patient may switch between available programs to select the program that provides the most suitable pain relief. Also, patient controller device 905 may enable patient 902 to control stimulation amplitude (for certain neurostimulation devices). For example, patient 902 may enter relevant information via one or more UI screens to control stimulation depending on patient's pain condition, activity level, etc. Further, IMD 903 may automatically employ different therapy settings when the patient 902 is asleep or when the patient is active. In other arrangements, IMD 903 may modify operations depending upon the intake or ingestion of pharmaceutical agents by patient 902. The patient 902 may enter relevant information via external device 905 to indicate such events.

In some embodiments, a historical record of all such interactions, modifications, reconfigurations, etc., with respect to IMD 903 and patient device 905 may be maintained and transmitted to servers 912. Clinicians may access such data either from servers 912 or from patent controller device 905 or from IMD 903 via a networked session with the patient 902 for facilitating/enhancing a remote care therapy consultation. For example, In a remote care therapy scenario, a clinician may not only be able to monitor and evaluate the patient based on a number of real-time variables such as, e.g., patient's facial features, physiological conditions, patient's responses to questions, etc., but also access the historical record of patient interactions with the IMD and utilize that information in providing appropriate therapy, wherein the communications between the patient and the clinician are advantageously protected. Accordingly, skilled artisans will recognize that the foregoing example scenarios 900A, 900B may be combined in a more comprehensive use case scenario where in-person therapy sessions (i.e., local sessions) and/or remote care therapy sessions may be effectuated at different times for purposes of an embodiment of the present disclosure after performing the requisite work flows relating to device registration and establishment of trusted associations as set forth in FIGS. 3-5 described hereinabove.

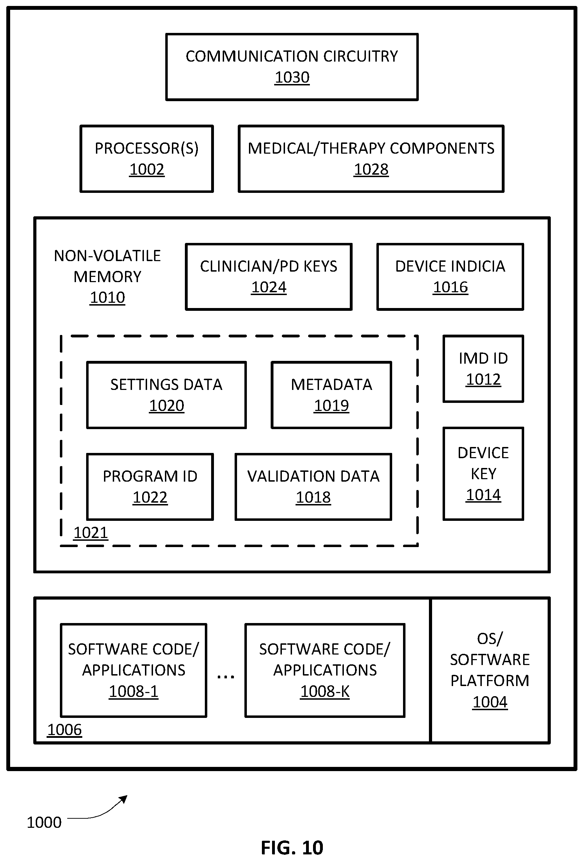

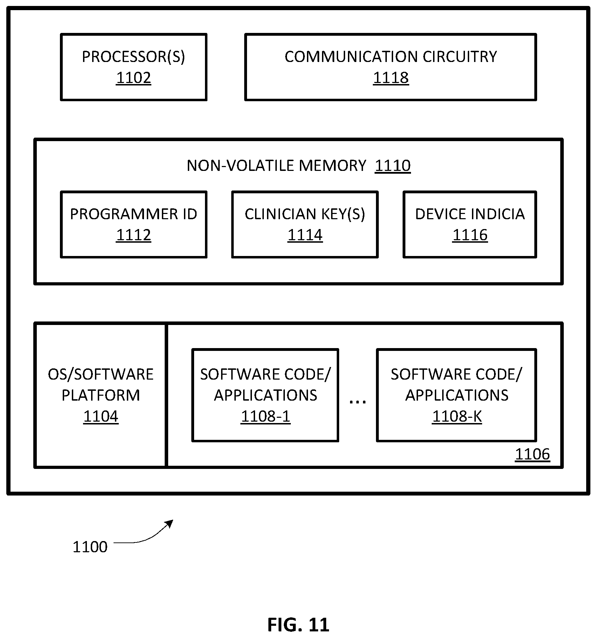

FIGS. 10 and 11 depict block diagrams of a patient medical device 1000 and a clinician device 1100, respectively, that may be configured to operate according to some example embodiments. In some implementations, at least portions of medical device 1000 may be arranged as an implantable medical device. In other arrangements, different portions of device 1000 may be configured so as to operate as a patient controller device. In general, device 1000 preferably includes one or more processors or controllers 1002 to control overall device operations, including operations relating to one or more medical therapy components 1028 to provide appropriate therapy to the patient and/or to monitor or measure one or more physiological conditions of the patient. In one arrangement, device 1000 may include suitable communications circuitry 1030 to conduct communication sessions with an external device (e.g., clinician device 1100 described in detail below and/or a patient device) after implantation using any known or heretofore unknown short-range communications technologies, e.g., involving communication protocols that may include but not limited to inductive communication protocols, BLE, NFC, Zigbee, UHF RFID, Bluetooth, and the like. In one arrangement, communications circuitry 1030 of device 1000 may also include circuitry to conduct communication sessions with networked devices over a network using appropriate technologies as set forth above in reference to FIG. 1. Further, device 1000 may include one or more OS platforms 1004 and one or more software applications 1008-1 to 1008-N depending on configuration, collectively referred to as device software environment 1006. Device 1000 may include one ore more memory modules 1010 including, e.g., non-volatile memory modules, to store executable instructions and data. In one arrangement, the stored data may include a device identifier 1012, one or more device keys 1014 and one or more device trust indicia 1016. For example, device key storage 1014 may store one of a pair of asymmetric encryption keys with the other key stored by a remote server, e.g., a PKI server or other server. The pair of keys for a given device 1000 may be used to securely create and employ validation data according to some embodiments. Although device identifier 1012 is shown as stored in memory 1010, device identifier 1012 may be retained elsewhere in device 1000. For example, many device components (e.g., processors, integrated circuits, wireless communication circuitry, and the like) include identifiers that are hard-encoded in the components and are readily retrievable. In one embodiment, the identifiers of such subcomponents may be used, taken alone or in some combination, as the medical device identifier in lieu of a value stored in memory of device 1000. Memory 1010 may also include storage for additional software code to control certain aspects of device 1000, which may include code or program instructions to implement operations relating to remote care therapy applications according to the embodiments herein.

Device 1000 may include one or more instances of programming data 1021 in memory 1010 that may be configured to define how device 1000 conducts therapeutic or medical operations according to some embodiments. In some embodiments, each instance of programming data 1021 may include a program identifier 1022. Also, each instance of programming data 1021 may include a field for device identifier data (not specifically shown), which may be compared against the device identifier 1012 to ensure that the programming data 1021 is intended for use by the specific device 1000. Further, each instance of programming data 1021 may include settings data 1020, e.g., comprising various device parameters that define the therapeutic or medical operations to be provided by device 1000. For example, in an embodiment involving a neurostimulation device for chronic pain, the settings data may include an electrode configuration for delivery of electrical pulses, a stimulation pattern identifier (tonic stimulation, burst stimulation, noise stimulation, and/or the like), pulse parameters, one or more frequency parameters, cycling parameters, timing parameters, and/or the like. Still further, each instance of programming data 1021 may be accompanied with its respective metadata 1019, which may include data that identifies the physician or clinician that created or programmed the settings data. In some embodiments, the metadata 1019 may include an identifier of the clinician programmer device that was used to create the settings data, the date of creation, the data of last modification, the physical location where programming occurred, and/or any other relevant data. Each instance of programming data 1021 may also include appropriate validation data 1018. For example, the validation data may be used by device 1000 to ensure that the settings data is intended for device 1000 and is properly authorized to control/configure the device operations. In some embodiments, validation data may be created using a checksum algorithm, a cryptographic hash function, and/or similar suitable processing. For example, some of the programming data 1021 may be represented by a plurality of characters in respective strings, wherein each character in sequence may be applied to a hash function or suitable function to generate an output hash value or similar value which may be verified by known checksum functions and/or modular sum operations. Furthermore, the checksum value or other relevant data may be encrypted with a suitable cryptographical key (e.g., the corresponding key of the key pair used for device 1000). The encrypted data may then be stored in device 1000 as the validation data in some embodiments. A separate storage area 1024 may be provided for securely storing applicable CD/PD keys used for securing communications with respect to remote care therapy sessions according to some embodiments.

Example clinician device 1100 may include one or more processors 1102, communications circuitry 1118 and one or more memory modules 1110, operative in association with one or more OS platforms 1104 and one or more software applications 1108-1 to 1108-K depending on configuration, cumulatively referred to as CD software environment 1106. Example OS platforms may include, without limitation, iOS, Android, Chrome OS, Blackberry OS, Ubuntu, Sailfish OS, Windows, Kai OS, etc. It will be realized that at least a portion of the software applications may include code or program instructions configured to execute various operations relative to device registration and establishment of trusted associations at a remote care session manager or server as set forth hereinabove. Memory modules 1110 may include non-volatile memory configured to store relevant data and software code (not specifically shown) to control overall operation of device 1100. Memory 1110 may include storage 1112 to store a programmer identifier (e.g., a serial number) of device 1100 used during programming sessions (e.g., local programming or remote session programming). Also, memory 1110 may store one or more clinician keys 1114 and one or more device indicia 1116 for use during programming and/or remote care therapy as discussed herein. Communications circuitry 1118 may include wireless and/or wireline communication capabilities, inductive communication capabilities, to effectuate IMD communications as well as networked communications as set forth hereinabove.

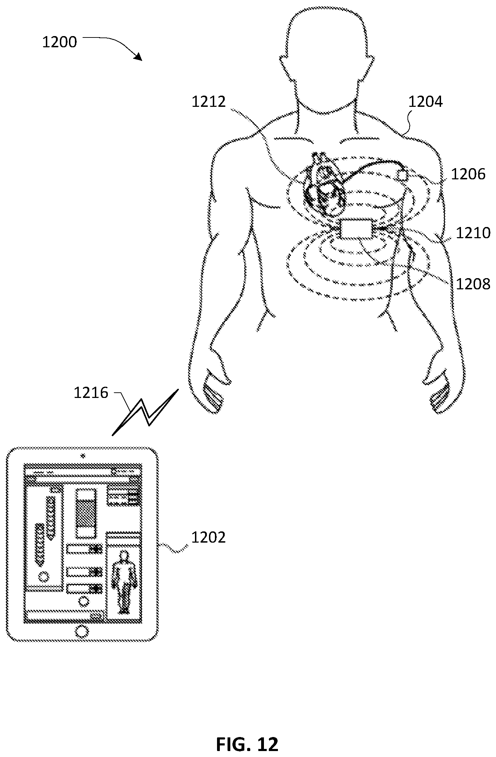

FIG. 12 depicts a simplified block diagram of a system 1200 for effectuating initial bonding associations between an external device and an IMD via a local bi-directional communication link when they are in proximity of each other for purposes of an example embodiment of the present invention. Broadly, system 1200 is representative of a scenario to provide a pairing and/or bonding procedure between two devices using a proximate triggering device, e.g., when a patient device (e.g., PC/PD 504) or a clinician device (e.g., CP/CD 304) is within the vicinity or presence of a patient having the IMD. In general operation, the triggering device may be configured to emit or transmit an activation field, which may be detected by the IMD. When the activation field is detected by the IMD, the IMD may enter or transition into a communication initialization mode corresponding to a preconfigured pairing and/or bonding procedure involving known or heretofore unknown communication protocols. For example, the pairing and/or bonding procedure may be defined by a wireless protocol (e.g., Bluetooth, BLE, ZigBee, etc.). The pairing and/or bonding procedure may include exchanging information to generate passkeys in both the IMD and an external device to establish a communication link. A technical effect of this embodiment is to strengthen pairing and/or bonding procedures for wireless protocols by providing proximity detection based on the triggering device and the activation field for the pairing and/or bonding procedure of available wireless protocols. Another technical effect of positioning the triggering device proximate to the IMD, thereby the patient, is awareness of the patient and proximity protection against the IMD initiating a communication link from an untrusted or unauthorized external device.

Illustratively, system 1200 is exemplified with an IMD 1206, a triggering device 1208 (e.g., a magnet, an inductive communication circuit, an NFC circuit, an electric motor, etc.), and an external device 1202, which may be operative as a clinician device or a patient device according to an embodiment. IMD 1206 may be implanted within a patient 1204 (e.g., proximate to the patient's heart 1212, proximate to the spinal cord, proximate to or within the brain, or proximate to some other tissue/organ of interest). Additionally or alternatively, IMD 1206 may have components that are external to the patient 1204. For example, IMD 1206 may include a wearable/external pulse generator (EPG) for providing appropriate stimulation pulses operative to be transmitted to one or more regions via IMD 1206.

The triggering device 1208 is operative to produce or generate an activation field 1210. IMD 1206 may be configured to detect the activation field 1210 when the IMD is passed through and/or placed within the activation field 1210. The activation field 1210 from the triggering device 1208 may comprise at least one of a magnetic field, NFC transmission, RFID transmission, an inductive telemetry signal, or a vibration scheme resulting in displacement of a position of the IMD 1206. The activation field 1210 may be defined by an effective distance or area from the triggering device 1208 where the activation field 1210 may be detected by IMD 1206. Optionally, the triggering device 102 may continually produce or emit the activation field 1210. For example, the activation field 1210 may include a magnetic field generated by a magnet of the triggering device 102. Additionally or alternatively, the activation field 1210 may be activated by a user. For example, the activation field 1210 may include an NFC transmission generated by an NFC circuit of the triggering device 1208, which is activated by the user when positioned proximate to the IMD 1206.

When the activation field 1210 is detected by the IMD 1206, the IMD 1206 may be programmed and/or configured to enter into a select communication initialization mode corresponding to the activation field 1210 generated from the triggering device 1208. The select communication initialization mode may comprise a subset of a plurality of communication initialization modes defined by the wireless protocol to establish a bi-directional communication link 1216 between the IMD 1206 and the external device 1202. For example, the communication initialization mode may correspond to a defined pairing and/or bonding procedure. Optionally, the communication initialization mode may comprise transitioning the IMD 1206 from a sleeping and/or power saving state by activating a radio frequency (RF) circuit (not specifically shown in this FIG.). Additionally or alternatively, the IMD 1206 may determine which communication initialization mode is selected based on a field characterization of the activation field, such as, e.g., a strength, frequency, phase and/or select characteristic value of the activation field 1210. For instance, the IMD 1206 may identify a magnetic field strength and/or flux of the activation field 1206 at a first value corresponding to a communication initialization mode for initiating the pairing and/or bonding procedure between the IMD 1206 and the external device 1202. In another example, the IMD 1206 may detect a magnetic field strength and/or flux of the activation field 1210 at a second value corresponding to a communication initialization mode for activating the RF circuit of the IMD 1206 for reestablishing the bi-directional communication link 1216 with a previously bonded and/or paired external device 1202.

The external device 1202 may be configured to establish the bi-directional communication link 1216 with the IMD 1206 so as to facilitate the external device 1202 to receive measurements from the IMD 1206 and/or to program or send instructions to the IMD 1206. As noted previously, the bi-directional communication link 1216 may use any standard wireless protocols. Additionally, some example embodiments may involve establishing the bi-directional communication link 1216 using certain healthcare-specific communications services including, e.g., Medical Implant Communication Service, Wireless Medical Telemetry Service, Medical Device Radiocommunications Service, Medical Data Service, etc. The external device 1202 may be located within a home of the patient, a hospital, an automobile, at an office of the patient/clinician, or the like. Accordingly, the example system 1200 may be implemented as a secure bonding procedure within the context of the message/work flows of FIGS. 4 and 5 for purposes of an example embodiment of the present invention. Whereas the IMD 1206 is particularly exemplified as a cardiac medical device in the system 1200, it should be understood that IMD 1206 is broadly representative of any type, category or class of implantable bioinstrumentation devices as noted previously. Additional details regarding the initiation of a bi-directional communication link between two devices using a proximate triggering device may be found in U.S. Pat. No. 9,288,614, entitled "SYSTEMS AND METHODS FOR INITIATING A COMMUNICATION LINK BETWEEN AN IMPLANTABLE MEDICAL DEVICE AND AN EXTERNAL DEVICE".

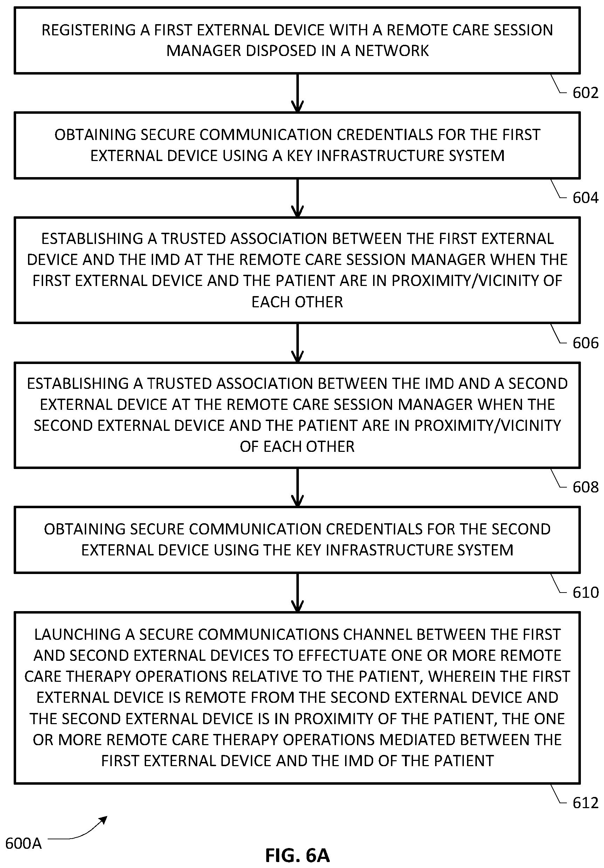

Turning attention to FIGS. 6A-6D, depicted therein are flowcharts illustrative of additional blocks, steps and/or acts that may be (re)combined in one or more arrangements with or without additional flowcharts of the present disclosure for facilitating remote care therapy according to one or more embodiments. Example process 600A of FIG. 6A may be commenced by registering a first external device with a remote care session manager disposed in a network (block 602). At block 604, secure communication credentials are obtained/generated for the first external device using a key infrastructure system. At block 606, a trusted association is established between the first external device and a patient's IMD at the remote care session manager when the first external device and the patient are in proximity of each other. At block 608, a trusted association is established between the IMD and a second external device at the remote care session manager when the second external device and the patient are in proximity of each other. At block 610, secure communication credentials are obtained/generated for the second external device using the key infrastructure system. Thereafter, a secure communications channel may be launched between the first and second external devices to effectuate one or more remote care therapy operations relative to the patient, wherein the first external device is remote from the second external device and the second external device is in proximity of the patient, the one or more remote care therapy operations being mediated between the first external device and the IMD of the patient, as set forth at block 612.

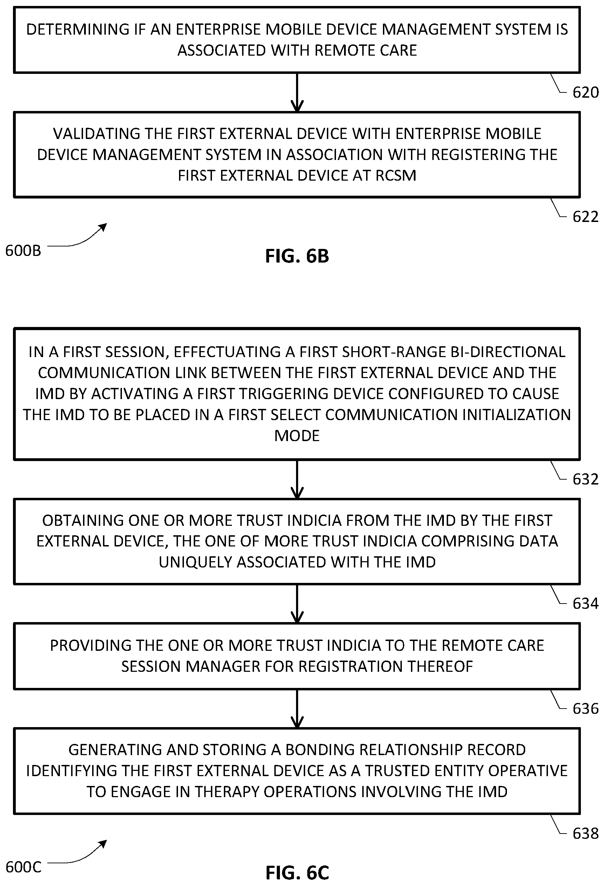

Example process 600B of FIG. 6B involves determining if an enterprise device management is associated with remote care therapy services, as set forth at block 620. If so, the first external device is validated with the enterprise device management system before registering the first external device in association with the remote care session manager (block 622). Example process 600C of FIG. 6C is representative of a trust association establishment process with respect to the first external device and the patient's IMD. In a first session, when the devices are in close proximity, a first short-range bi-directional communication link may be effectuated between the first external device and the IMD by activating a first triggering device configured to cause the IMD to be placed in a first select communication initialization mode (block 632). At block 634, one or more trust indicia are retrieved or otherwise obtained from the IMD by the first external device, wherein the one or more trust indicia comprises data uniquely associated with the IMD. As noted previously, various pieces of data or information may be provided to operate as such trust indicia. At block 636, at least a portion of trust indicia may be transmitted to the remote care session manager for registration of the IMD therewith. At block 638, a bonding relationship record may be created and stored that includes suitable information for identifying or otherwise indicating the first external device as a trusted entity operative to engage in therapy operations involving the IMD.

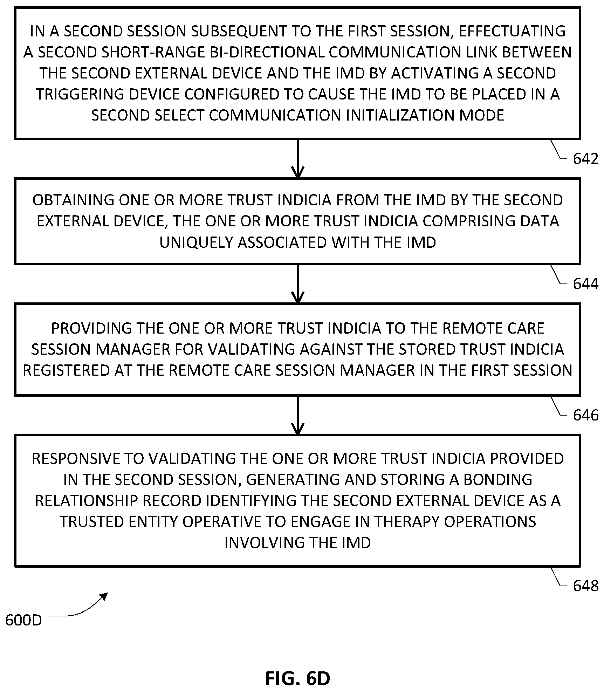

Example process 600D of FIG. 6D is representative of a trust association establishment process with respect to the second external device and the patient's IMD. In a second session subsequent to the first session, a second short-range bi-directional communication link may be effectuated between the second external device and the IMD by activating a second triggering device configured to cause the IMD to be placed in a second select communication initialization mode (block 642). In some example embodiments, different types of triggering devices and/or technologies may be used with respect to the respective secure bonding processes of the first and second external devices. At block 644, one or more trust indicia are retrieved or otherwise obtained from the IMD by the second external device, wherein the one or more trust indicia comprises data uniquely associated with the IMD. At block 646, at least a portion of the trust indicia is transmitted to the remote care session manager for validating against the stored trust indicia registered at the remote care session manager in the first session. Responsive to validating the one or more trust indicia provided in the second session, a bonding relationship record may be generated and stored at the remote care session manager for identifying/indicating the second external device as a trusted entity operative to engage in therapy operations involving the IMD.

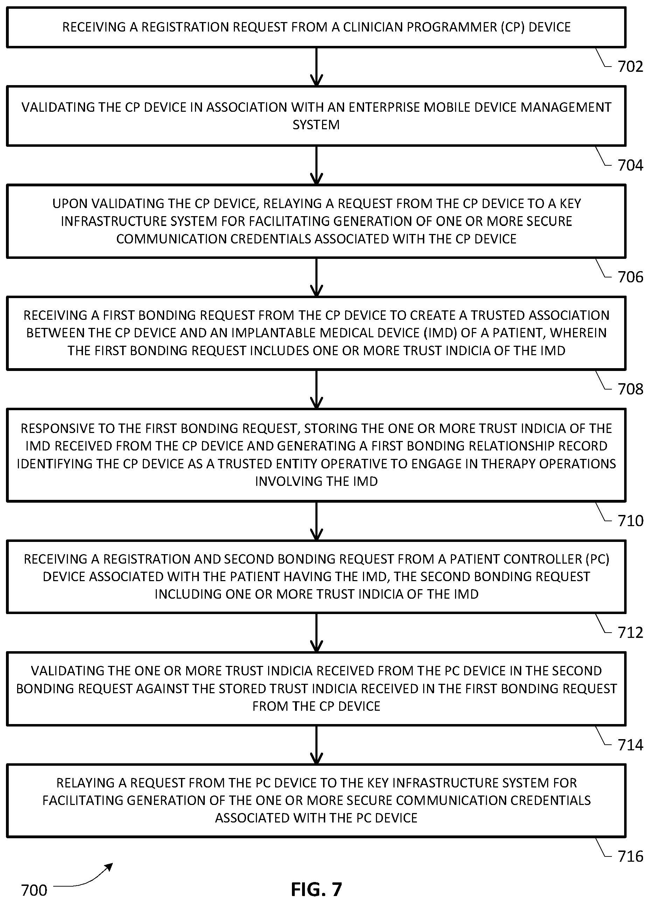

FIG. 7 depicts a flowchart illustrative of a process 700 comprising various blocks, steps and/or acts that may take place at a remote care session manager for purposes an example embodiment of the present invention. At block 702, a registration request is received from a clinician programmer (CP) device (i.e., a first external device). At block 704, the CP device is validated by the remote care session manager, preferably or optionally in association with an enterprise mobile device management system. At block 706, upon validating the CP device, a request from the CP device is relayed to a key infrastructure system for facilitating generation of one or more secure communication credentials associated with the CP device. At block 708, a first bonding request is received from the CP device to create a trusted association between the CP device and an IMD of a patient, wherein the first bonding request includes one or more trust indicia of the IMD. Responsive to the first bonding request, the remote care session manager stores the one or more trust indicia of the IMD received from the CP device and generates a first bonding relationship record identifying the CP device as a trusted entity operative to engage in therapy operations involving the IMD (block 710). At block 712, a registration and second bonding request is received from a patient controller (PC) device (e.g., a second external device) associated with the patient having the IMD, the second bonding request including one or more trust indicia of the IMD. At block 714, the remote care session manager is operative to validate the one or more trust indicia received from the PC device in the second bonding request against the stored trust indicia received in the first bonding request from the CP device. Thereafter, upon successful validation, a request from the PC device is relayed to the key infrastructure system for facilitating generation of one or more secure communication credentials associated with the PC device (at block 716).

Skilled artisans will recognize that some of the blocks, steps and/or acts set forth above may take place at different times (i.e., asynchronously), and possibly with intervening gaps of time and/or at different places, as noted elsewhere in the present patent application. Accordingly, it should be appreciated that the process flows (as well as the message/work flows set forth in FIGS. 3-5) may be interleaved with one or more sub-processes comprising other IMD-patient or IMD-clinician interactions (e.g., local therapy sessions), which may alter, modify or otherwise reconfigure the processes and message/work flows in some embodiments.

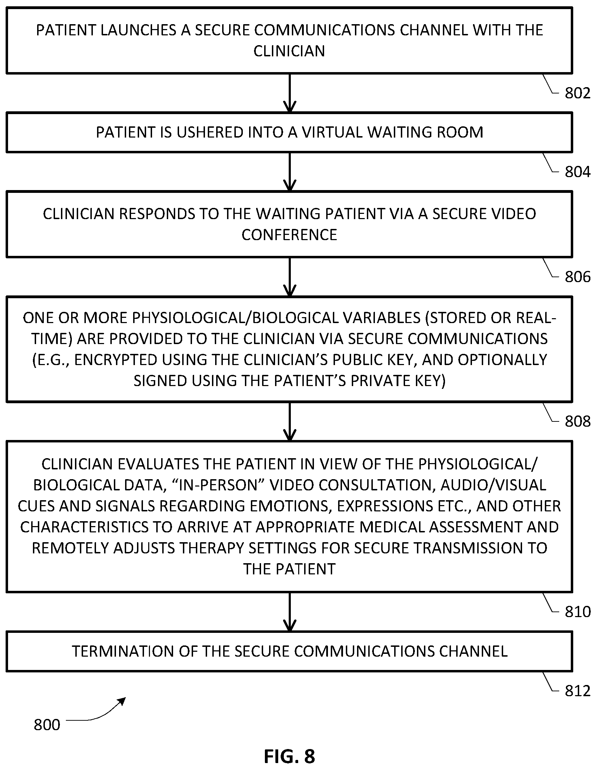

FIG. 8 depicts a flowchart illustrative of a process flow 800 with respect to a remote care therapy use case scenario according to an example embodiment of the present invention. At block 802, a patient launches a therapy application executing on the patient controller/device to initiate a secure communications channel with a remote clinician (e.g., by selecting a "Remote Care" option from a pull-down menu, clicking on an icon on the UI display screen, or via a voice command, etc.). In one embodiment, the patient may be ushered into a virtual waiting room, realized in a UI screen window of the patient controller/device (block 804). At block 806, the clinician responds to the waiting patient, e.g., via a secure video conference or chat room, audio/video messaging application, etc. At block 808, one or more physiological/biological variables (stored or real-time) may be provided to the clinician via secure communications (e.g., facilitated via a Transport Layer Security (TLS)-based path based on the encryption keys). In some embodiments, data may be encrypted using the clinician's public key, and optionally signed by using the patient's private key. At block 810, the clinician evaluates the patient in view of the physiological/biological data, "in-person" video consultation, audio/visual cues and signals regarding patient's facial expressions, hand movement/tremors, walking, gait, ambulatory status/stability, and other characteristics to arrive at appropriate medical assessment and remotely adjusts therapy settings for secure transmission to the patient device, which may be securely transmitted via encrypted communications (e.g., encrypted using the patient's public key, and optionally signed by using the clinician's private key). In an illustrative scenario, a remote clinician proxy or agent may be executed at or in association with the patient controller/device upon launching a remote session, wherein the proxy/agent is operative to effectuate or otherwise mediate the transmission of any therapy settings to the patient's IMD, either in real-time or at some point in the future depending upon programmatic control. After completing the video consultation, the secure communications channel may be terminated, e.g., either by the clinician and/or the patient, as set forth at block 812.

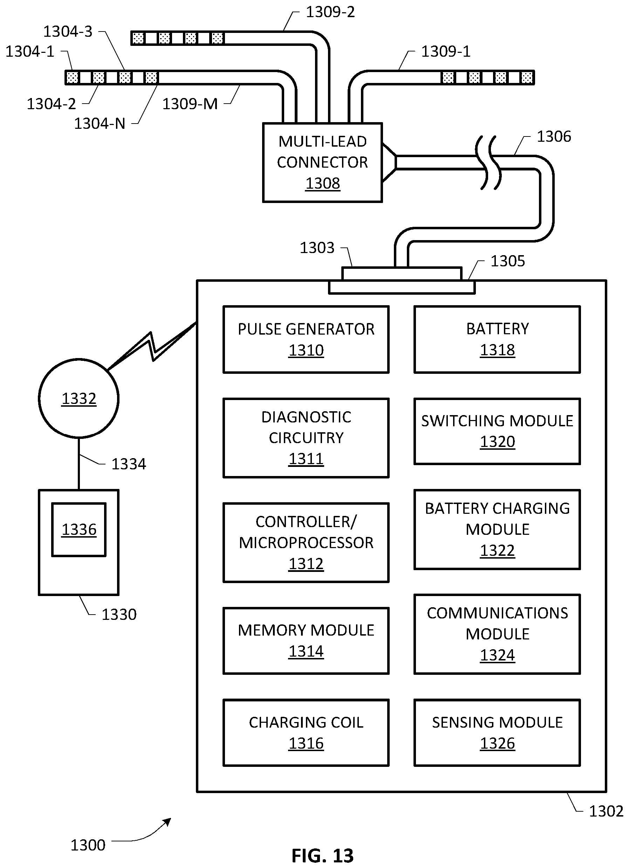

Turning to FIG. 13, depicted therein is a block diagram of a therapy system 1300 having an IMD 1302 that may be configured for a remote therapy application for purposes of an example embodiment of the present invention. In general, therapy system 100 may be adapted to stimulate spinal cord tissue, peripheral nerve tissue, deep brain tissue, DRG tissue, cortical tissue, cardiac tissue, digestive tissue, pelvic floor tissue, or any other suitable biological tissue of interest within a patient's body, as noted above. IMD 1302 may include a diagnostic circuit module 1311 adapted to effectuate various diagnostics with respect to the state/condition of one or more stimulation electrodes and sensing electrodes of an implantable lead system as well as other bio/physiological sensors integrated or otherwise operative with IMD 1302. In one example embodiment, IMD 1302 may be implemented as having a metallic housing or can that encloses a controller/processing block or module 1312, pulse generating circuitry 1310, a charging coil 1316, a battery 1318, a far-field and/or near field communication block or module 1324, battery charging circuitry 1322, switching circuitry 1320, sensing circuitry 1326, a memory module 1314, and the like. Controller/processor module 1312 typically includes a microcontroller or other suitable processor for controlling the various other components of IMD 1302. Software/firmware code may be stored in memory 1314 of IMD 102, which may be integrated with the controller/processor module 1312, and/or other suitable application-specific storage components (not particularly shown in this FIG.) for execution by the microcontroller or processor 1312 and/or other programmable logic blocks to control the various components of the device for purposes of an embodiment of the present patent disclosure.

In one arrangement, IMD 1302 may be configured to couple to one or more stimulation leads 1309-1 to 1309-M using an implantable multi-lead connector 1308 operative to receive corresponding stimulation leads 1309-1 to 1309-M at their respective proximal ends for securely engaging and providing electrical connectivity with respect to each stimulation lead's distal end having a plurality of stimulation electrodes. By way of illustration, stimulation lead 1309-M is exemplified with stimulation electrodes 1304-1 to 1304-N, which may be implanted near or adjacent to the patient's target tissue. Stimulation leads 1309-1 to 1309-M may comprise percutaneous leads, paddle leads, etc., wherein the electrodes may comprise ring electrodes, segmented or split electrodes, planar electrodes, and the like. Preferably, a single lead cable 1306 may be provided for electrically connecting the multi-lead connector 1308 to IPG 102 via a suitable connector interface or socket 1303 that may be mated to an interface receptacle or header portion 1305 of IMD 1302. In general, electrical pulses are generated by the pulse generating circuitry 1310 under the control of processing block 1312, and are provided to the switching circuitry 1320 that is operative to selectively connect to electrical outputs of IMD 1302, wherein one or more stimulation leads 1309-1 to 1309-M and/or one more stimulation electrodes 1304-1 to 1304-N per each lead may be energized according to a therapy protocol, e.g., by the patient (via a local session) and/or a clinician (via a local or remote session) using a corresponding external device, e.g., device 1330. Further, external device 1330 may also be configured to charge/recharge the battery 1318 of IMD 1302 (although a separate recharging device could alternatively be employed), in addition to accessing memory 1314, and/or (re)programming IMD 1302 with respect to the stimulation set parameters including pulsing specifications while implanted within the patient. In alternative embodiments, however, separate programmer devices may be employed for charging and/or programming the IMD 1302 device and/or any programmable components thereof. A connector or "wand" 1334 may be electrically coupled to the external device 1330 through suitable electrical connectors (not specifically shown), which may be electrically connected to a telemetry component 1332 (e.g., inductor coil, RF transceiver, etc.) at the distal end of wand 1334 through respective communication links that allow bi-directional communication with IMD 1302 in a local session scenario. Optionally, in some embodiments, the wand 1334 may comprise one or more temperature sensors for use during charging operations.

In an example scenario, a user (e.g., a doctor, a medical technician, or the patient) may initially communicate with IPG 1302 by placing the wand 1334 proximate to the IMD 1302. Preferably, the placement of the wand 1334 allows the telemetry system to be aligned with the far-field and/or near field communication circuitry 1324 of IMD 1302 for facilitating bonding operations, programming operations, and the like. The external device 1330 preferably provides one or more user interfaces 1336 (e.g., touch screen, keyboard, mouse, buttons, scroll wheels or rollers, or the like), allowing the user to operate IMD 1302. The external device 1330 may be controlled by the user through the user interface 1336, allowing the user to interact with IMD 102, including, e.g., effectuating programmatic control for facilitating diagnostic measurements, dynamically configuring electrodes for different therapy schemes, etc. Further, the user interface 1336 may permit the user to move electrical stimulation along and/or across one or more of the leads 1309-1 to 1309-M using different lead electrode combinations selected from electrodes, for example, as described in U.S. Patent Application Publication No. 2009/0326608, entitled "METHOD OF ELECTRICALLY STIMULATING TISSUE OF A PATIENT BY SHIFTING A LOCUS OF STIMULATION AND SYSTEM EMPLOYING THE SAME", . Optionally, the user interface 1336 may permit the user to designate which electrodes 1304-1 to 1304-N of a particular lead are to stimulate (e.g., emit current pulses, in an anode state, in a cathode state), or not selected to stimulate (i.e., remain inactive or floating, i.e., "unused"), with respect to a potential stimulation target, to measure/sense tissue electrical parameters, or the like. As used herein "stimulation" refers to the application of an electrical signal to a target body tissue, regardless of the effect that signal is intended to produce. Additionally or alternatively, the external device 1330 may access or download the electrical measurements from the memory 1314 acquired by the sensing circuitry 1326.

In some implementations, the external device 1330 may permit operation of IMD 1302 according to one or more SCS programs or therapy applications to treat the patient. Each SCS program may include one or more sets of stimulation parameters of the pulse including pulse amplitude, stimulation level, pulse width, pulse frequency or inter-pulse period, pulse repetition parameter (e.g., number of times for a given pulse to be repeated for respective stimulation sets or stimsets during execution of program), biphasic pulses, monophasic pulses, etc. IMD 1302 modifies its internal parameters in response to the control signals from the external device 1330 to vary the stimulation characteristics of the stimulation therapy transmitted through the lead system 1309-1 to 1309-M to the tissue of the patient. Example neurostimulation (NS) systems, stimsets, and multi-stimset programs are set forth in U.S. Pat. No. 7,228,179, entitled "METHOD AND APPARATUS FOR PROVIDING COMPLEX TISSUE STIMULATION PATTERNS", and International Patent Publication Number WO 2001/093953, entitled "NEUROMODULATION THERAPY SYSTEM".

Skilled artisans will recognize that at least some functionalities and components of IMD 1302 and at least some functionalities and components of device 1000 of FIG. 10 may be combined for purposes of an example embodiment involving remote care therapy. Likewise, at least some functionalities and components of device 1330 may be combined with at least some functionalities and components of device 1100 of FIG. 11 in an embodiment involving remote care therapy. Accordingly, various types of therapy operations exemplified above may be effectuated by a clinician via a secure remote session involving the clinician's device 1330, patient's device (not shown in this FIG.) and the patient's IPG/IMD 1302 according to example embodiments of the present invention, which in some use case scenarios may involve one or more message/work flows and processes set forth previously in the present patent application.

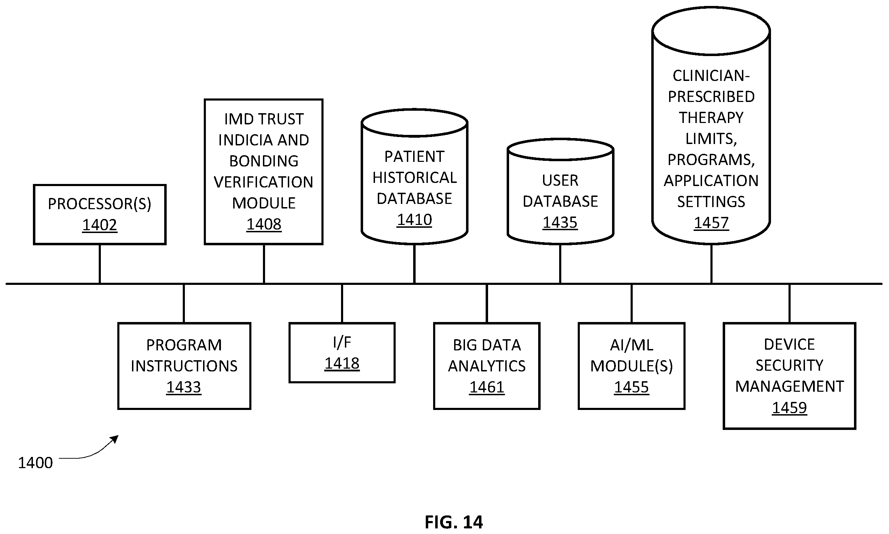

FIG. 14 depicts a block diagram involving a plurality of modules that may be configured as a computer-implemented network node, apparatus or platform 1400 operative at a service provider network, datacenter facility, etc., which may include remote care session management functionalities for purposes of an example embodiment of the present invention. One or more processors 1402 may be operatively coupled to various modules that may be implemented in persistent memory for executing suitable program instructions or code portions (e.g., code portion 1433) with respect to effectuating remote device management, establishment of trusted associations, interfacing with encryption key management systems and the like, preferably in association with one or more other modules and/or databases of the platform 1400. As skilled artisans will recognize, the program instructions or code portions provided as part of the platform 1400 may be configured in a number of ways operative to execute one or more process flows described hereinabove. An IMD trust indicia verification module 1408 may be provided to create, maintain and store bonding associations between IMDs and clinician devices, which may be used for verification/bonding of patient controllers/devices. In an example embodiment, a patient history database 1410 and a user database 1435 may also provided wherein the database(s) are operative to maintain and store patients' historical data relating to therapies, biophysiological conditions monitoring, etc., as well as patient profile data, clinician profile data, and the like. In some arrangements, clinician-prescribed therapy settings, stimulation application programs, stimulation level thresholds and limits, etc., may be stored in a database 1457, e.g., on a patient-by-patient basis and/or IMD-by-IMD basis, which may be made available to remote clinicians engaged in a particular therapy session. Also, a security management module 1459 may be provided as part of the platform 1400 for interfacing and proxying clinician devices and patient devices with respect to corresponding key infrastructure systems.

In a further embodiment, the platform 1400 may include one or more machine language modules 1455 operative in association with "Big Data" analytics module(s) 1461 for facilitating intelligent data-mining operations and adaptive learning of therapy operations based on patients' historical therapy data, biophysiological data, and other relevant data that may be sourced from external entities. In the context of some example remote care therapy use case scenarios, "Big Data" may be used as a term for a collection of data sets so large and complex that it becomes virtually impossible to process using conventional database management tools or traditional data processing applications. Challenges involving "Big Data" may include capture, curation, storage, search, sharing, transfer, analysis, and visualization, etc. Because "Big Data" available with respect to patients' diagnostic data, user profile data, real-time monitoring of patients' biophysiological conditions, Internet-of Things (IoT)-based sensor data gathered from patients and respective ambient surroundings, etc., can be on the order of several terabytes to petabytes to exabytes, it becomes exceedingly difficult to work with using most relational database management systems for optimizing, ranking, indexing, cross-correlating test/measurement data and status data in typical environments. Accordingly, in one arrangement, the Big Data analytics module 1461 and appropriate therapy domain knowledgebase(s) (not specifically shown in this FIG.) may be implemented in a machine learning framework that is optimized for storage and large-scale processing of data sets on clusters of commodity hardware. Skilled artisans will further recognize that an example machine learning framework may be implemented using one or more ML techniques, processes or algorithms and models (e.g., neural networks) as well as rule-based systems. In general, a variety of techniques such as, e.g., artificial intelligence (AI), convolutional neural networks (CNNs), fuzzy logic learning, pattern recognition, support vector machines (SVMs), support vector networks (SVNs) and related techniques, may be employed in a suitable combination or sub-combination with respect to effectuating an AI/ML-enhanced remote care therapy use case scenario involving the platform 1400. Further, in view of the flexible architecture of the platform 1400, one or more network interfaces (I/F) 1418 may be provided for interfacing with various external nodes or infrastructural elements, e.g., involving access and/core communications networks, external databases, cryptographic key infrastructure nodes, business support system nodes, third-party healthcare provider networks, and the like.

In the above-description of various embodiments of the present disclosure, it is to be understood that the terminology used herein is for the purpose of describing particular embodiments only and is not intended to be limiting of the invention. Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of this specification and the relevant art and may not be interpreted in an idealized or overly formal sense expressly so defined herein.

At least some example embodiments are described herein with reference to one or more circuit diagrams/schematics, block diagrams and/or flowchart illustrations. It is understood that such diagrams and/or flowchart illustrations, and combinations of blocks in the block diagrams and/or flowchart illustrations, can be implemented by any appropriate circuitry configured to achieve the desired functionalities. Accordingly, example embodiments of the present disclosure may be embodied in hardware and/or in software (including firmware, resident software, micro-code, etc.) operating in conjunction with suitable processing units or microcontrollers, which may collectively be referred to as "circuitry," "a module" or variants thereof. An example processing unit or a module may include, by way of illustration, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Array (FPGA) circuits, any other type of integrated circuit (IC), and/or a state machine, as well as programmable system devices (PSDs) employing system-on-chip (SoC) architectures that combine memory functions with programmable logic on a chip that is designed to work with a standard microcontroller. Example memory modules or storage circuitry may include volatile and/or non-volatile memories such as, e.g., random access memory (RAM), electrically erasable/ programmable read-only memories (EEPROMs) or UV-EPROMS, one-time programmable (OTP) memories, Flash memories, static RAM (SRAM), etc.

Further, in at least some additional or alternative implementations, the functions/acts described in the blocks may occur out of the order shown in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Furthermore, although some of the diagrams include arrows on communication paths to show a primary direction of communication, it is to be understood that communication may occur in the opposite direction relative to the depicted arrows. Finally, other blocks may be added/inserted between the blocks that are illustrated.

It should therefore be clearly understood that the order or sequence of the acts, steps, functions, components or blocks illustrated in any of the flowcharts depicted in the drawing Figures of the present disclosure may be modified, altered, replaced, customized or otherwise rearranged within a particular flowchart, including deletion or omission of a particular act, step, function, component or block. Moreover, the acts, steps, functions, components or blocks illustrated in a particular flowchart may be inter-mixed or otherwise inter-arranged or rearranged with the acts, steps, functions, components or blocks illustrated in another flowchart in order to effectuate additional variations, modifications and configurations with respect to one or more processes for purposes of practicing the teachings of the present patent disclosure.

Although various embodiments have been shown and described in detail, the claims are not limited to any particular embodiment or example. None of the above Detailed Description should be read as implying that any particular component, element, step, act, or function is essential such that it must be included in the scope of the claims. Reference to an element in the singular is not intended to mean "one and only one" unless explicitly so stated, but rather "one or more." Moreover, the terms "first," "second," and "third," etc. employed in reference to elements or features are used merely as labels, and are not intended to impose numerical requirements, sequential ordering or relative degree of significance or importance on their objects.

## Claims

1. A method (600A) for facilitating remote care therapy for a patient (102, 902) having an implantable medical device, IMD (103, 903), the method (600A) comprising:
registering (602) a first external device (130, 904) with a remote care session manager (120) disposed in a network (118);
obtaining (604) secure communication credentials for the first external device (130, 904) using a key infrastructure system (122);
establishing (606) a trusted association between the first external device (130, 904) and the IMD (103, 903) at the remote care session manager (120) when the first external device (130, 904) and the patient (102, 902) are in proximity of each other;
establishing (608) a trusted association between the IMD (103, 903) and a second external device (104, 905) at the remote care session manager (120) when the second external device (104, 905) and the patient (102, 902) are in proximity of each other;
obtaining (610) secure communication credentials for the second external device (104, 905) using the key infrastructure system (122); and
launching (612), based on the trusted association between the first external device and the IMD and the trusted association between the IMD and the second external device, a secure communications channel between the first and second external devices (130, 904; 104, 905) to effectuate one or more remote care therapy operations relative to the patient (102, 902), wherein the first external device (130, 904) is remote from the second external device (104, 905) and the second external device (104, 905) is in proximity of the patient (102, 902), the one or more remote care therapy operations mediated between the first external device (130, 904) and the IMD (103, 903) of the patient (102, 902).

2. The method (600A, 600B) as recited in claim 1, further comprising validating (622) the first external device (130, 904) with an enterprise mobile device management system (307) in association with registering the first external device (130, 904).

3. The method (600A-600C) as recited in claim 2, wherein establishing the trusted association between the first external device (130, 904) and the IMD (103, 903) comprises performing following acts in a first session:
effectuating (632) a first short-range bi-directional communication link between the first external device (130, 904) and the IMD (103, 903) by activating a first triggering device configured to cause the IMD (103, 903) to be placed in a first select communication initialization mode;
obtaining (634) one or more trust indicia from the IMD (103, 903) by the first external device (130, 904), the one or more trust indicia comprising data uniquely associated with the IMD (103, 903);
providing (636) the one or more trust indicia to the remote care session manager (120) for registration therewith; and
generating and storing (638) a bonding relationship record identifying the first external device (130, 904) as a trusted entity operative to engage in therapy operations involving the IMD (103, 903).

4. The method (600A-600D) as recited in claim 3, wherein establishing the trusted association between the second external device (104, 905) and the IMD (103, 903) comprises performing following acts in a second session subsequent to the first session:
effectuating (642) a second short-range bi-directional communication link between the second external device (104, 905) and the IMD (103, 903) by activating a second triggering device configured to cause the IMD (103, 903) to be placed in a second select communication initialization mode;
obtaining (644) one or more trust indicia from the IMD (103, 903) by the second external device (104, 905), the one or more trust indicia comprising data uniquely associated with the IMD (103, 903);
providing (646) the one or more trust indicia to the remote care session manager (120) for validating against the stored trust indicia registered at the remote care session manager (120) in the first session;
responsive to validating the one or more trust indicia provided in the second session, generating and storing (648) a bonding relationship record identifying the second external device (104, 905) as a trusted entity operative to engage in therapy operations involving the IMD (103, 903).

5. The method (600A-600D) as recited in claim 4, wherein the one or more trust indicia comprise at least one of a serial number of the IMD (103, 903), a read-out of an on-board real-time clock, RTC, of the IMD (103, 903), a timestamp indicating when the IMD (103, 903) is implanted in the patient (102, 902), a device key stored in the IMD (103, 903), a device identifier associated with the IMD (103, 903), one or more patient biometric data stored in the IMD (103, 903), and one or more program identifiers associated with therapy programming data stored in the IMD (103, 903).

6. The method (600A-600D) as recited in claim 5, wherein the first session is effectuated by a clinician (906) operating the first external device (130, 904) and the second session is effectuated by the patient (102, 902) operating the second external device (104, 905) when the clinician (906) and the patient (102, 902) are engaged in an in-person initial consultation with respect to initializing at least one of respective first and second external devices (130, 904; 104, 905) for facilitating the one or more remote care therapy operations mediated via the IMD (103, 903).

7. The method (600A-600D) as recited in claim 6, wherein the key infrastructure system (122) comprises a public key infrastructure, PKI, system configured to provide a first key pair including a first public key and a first private key, the first key pair associated with at least one of the first external device (130, 904) and the clinician (906), and further wherein a first public key certificate is generated responsive to a first certificate signing request, CSR, emanating from the first external device (130, 904).

8. The method (600A-600D) as recited in claim 7, wherein the PKI system is configured to provide a second key pair including a second public key and a second private key, the second key pair associated with at least one of the second external device (104, 905) and the patient (102, 902), and further wherein a second public key certificate is generated responsive to a second certificate signing request, CSR, emanating from the second external device (104, 905).

9. A system (100) for facilitating remote care therapy for a patient (102), the system (100) comprising:
a first external device (130) associated with a clinician (138);
a second external device (104) associated with the patient (102);
an implantable medical device, IMD, (103) implanted in the patient (102), the IMD (103) supporting a therapy application configured to be programmable by at least one of the clinician (138) operating the first external device (130) and the patient (102) operating the second external device (104); and
a remote care session manager (120) supporting a first trusted association between the first external device (130) and the IMD (103) and a second trusted association between the second external device (104) and the IMD (103),
wherein the first external device (130) is configured to:
register with the remote care session manager (120);
obtain a first set of secure communication credentials using a key infrastructure system (122) in association with the remote care session manager (120), the first set of secure communication credentials operative for effectuating secure communications with the second external device (104); and
generate a first bonding request to the remote care session manager (120) with respect to the first trusted association effectuated when the first external device (130) and the patient (102) are in proximity of each other, wherein the first bonding request includes one or more trust indicia of the IMD (103) for registration and storage at the remote care session manager (120),
wherein the second external device (104) is configured to:
register with the remote care session manager (120);
generate a second bonding request to the remote care session manager (120) with respect to the second trusted association effectuated when the second external device (104) and the patient (102) are in proximity of each other, wherein the second bonding request includes one or more trust indicia of the IMD (103) provided for validation at the remote care session manager (120) against the stored trust indicia received in the first bonding request from the first external device (130); and
upon successfully effectuating the second trusted association, obtain a second of secure communication credentials using the key infrastructure system (122) in association with the remote care session manager (120), the second set of secure communication credentials operative for effectuating secure communications with the first external device (130), and
wherein at least one of the first and second external devices (130, 104) is operative to launch, based on the trusted association between the first external device and the IMD and the trusted association between the IMD and the second external device, a secure communications channel to effectuate one or more remote care therapy operations relative to the patient (102), the secure communication channel effectuated via a remote communication connection between the first and second external devices (130, 104) when the first external device (130) is remote from the second external device (104) and the second external device (104) is in proximity of the patient (102), the one or more remote care therapy operations mediated between the first external device (130) and the IMD (103) of the patient (102).

10. The system (100) as recited in claim 9, wherein the remote care session manager (120) is configured to validate the first external device (130) with an enterprise mobile device management system (307) in association with registering the first external device (130).

11. The system (100) as recited in claim 10, wherein the one or more trust indicia comprise at least one of a serial number of the IMD (103), a read-out of an on-board real-time clock, RTC, of the IMD (103), a timestamp indicating when the IMD (103) is implanted in the patient (102), a device key stored in the IMD (103), a device identifier associated with the IMD (103), one or more patient biometric data stored in the IMD (103), and one or more program identifiers associated with therapy programming data stored in the IMD (103).

12. The system (100) as recited in claim 11, wherein the first bonding request is effectuated by the clinician (138) operating the first external device (130) and the second bonding request is effectuated by the patient (102) operating the second external device (104) when the clinician (138) and the patient (102) are engaged in an in-person initial consultation with respect to initializing at least one of respective first and second external devices (130, 104) for facilitating the remote care therapy operations mediated via the IMD (103).

13. The system (100) as recited in claim 9, wherein the IMD (103) is configured to support at least one of a spinal cord stimulation, SCS, therapy, a neuromuscular stimulation therapy, a dorsal root ganglion, DRG, stimulation therapy, a deep brain stimulation, DBS, therapy, a cochlear stimulation therapy, a drug delivery therapy, a cardiac pacemaker therapy, a cardioverter-defibrillator therapy, a cardiac rhythm management, CRM, therapy, an electrophysiology, EP, mapping and radio frequency, RF, ablation therapy, an electroconvulsive therapy, ECT, a repetitive transcranial magnetic stimulation, rTMS, therapy, a vagal nerve stimulation, VNS, therapy, and one or more physiological condition monitoring applications.

## Patentansprüche

1. Verfahren (600A) zum Ermöglichen einer Fernbetreuungstherapie für einen Patienten (102, 902) mit einer implantierbaren medizinischen Vorrichtung, IMD (103, 903), wobei das Verfahren (600A) Folgendes umfasst:
Registrieren (602) einer ersten externen Vorrichtung (130, 904) bei einem Fernbetreuungs-Sitzungsmanager (120), der in einem Netzwerk (118) angeordnet ist;
Erhalten (604) von sicheren Kommunikationsberechtigungsdaten für die erste externe Vorrichtung (130, 904) unter Verwendung eines Schlüsselinfrastruktursystems (122);
Einrichten (606) einer vertrauenswürdigen Zuordnung zwischen der ersten externen Vorrichtung (130, 904) und der IMD (103, 903) am Fernbetreuungs-Sitzungsmanager (120), wenn sich die erste externe Vorrichtung (130, 904) und der Patient (102, 902) in der Nähe zueinander befinden;
Einrichten (608) einer vertrauenswürdigen Zuordnung zwischen der IMD (103, 903) und einer zweiten externen Vorrichtung (104, 905) am Fernbetreuungs-Sitzungsmanager (120), wenn sich die zweite externe Vorrichtung (104, 905) und der Patient (102, 902) in der Nähe zueinander befinden;
Erhalten (610) von sicheren Kommunikationsberechtigungsdaten für die zweite externe Vorrichtung (104, 905) unter Verwendung des Schlüsselinfrastruktursystems (122); und
Aufbauen (612), basierend auf der vertrauenswürdigen Zuordnung zwischen der ersten externen Vorrichtung und der IMD und der vertrauenswürdigen Zuordnung zwischen der IMD und der zweiten externen Vorrichtung, eines sicheren Kommunikationskanals zwischen der ersten und der zweiten externen Vorrichtung (130, 904; 104, 905), um eine oder mehrere Operationen der Fernbetreuungstherapie in Bezug auf den Patienten (102, 902) auszuführen, wobei sich die erste externe Vorrichtung (130, 904) entfernt von der zweiten externen Vorrichtung (104, 905) befindet und sich die zweite externe Vorrichtung (104, 905) in der Nähe des Patienten (102, 902) befindet, wobei die eine oder die mehreren Operationen der Fernbetreuungstherapie zwischen der ersten externen Vorrichtung (130, 904) und der IMD (103, 903) des Patienten (102, 902) vermittelt werden.

2. Verfahren (600A, 600B) nach Anspruch 1, ferner umfassend das Validieren (622) der ersten externen Vorrichtung (130, 904) mit einem Managementsystem für mobile Vorrichtungen für Unternehmen (307) in Zuordnung zum Registrieren der ersten externen Vorrichtung (130, 904).

3. Verfahren (600A-600C) nach Anspruch 2, wobei das Einrichten der vertrauenswürdigen Zuordnung zwischen der ersten externen Vorrichtung (130, 904) und der IMD (103, 903) das Durchführen der folgenden Handlungen in einer ersten Sitzung umfasst:
Herstellen (632) einer ersten bidirektionalen Kurzstrecken-Kommunikationsverbindung zwischen der ersten externen Vorrichtung (130, 904) und der IMD (103, 903) durch Aktivieren einer ersten Auslösevorrichtung, die dazu konfiguriert ist, zu veranlassen, dass die IMD (103, 903) in einen ersten ausgewählten Kommunikationsinitialisierungsmodus versetzt wird;
Erhalten (634) eines oder mehrerer Vertrauensmerkmale von der IMD (103, 903) durch die erste externe Vorrichtung (130, 904), wobei das eine oder die mehreren Vertrauensmerkmale Daten umfassen, die der IMD (103, 903) eindeutig zugeordnet sind;
Bereitstellen (636) des einen oder der mehreren Vertrauensmerkmale an den Fernbetreuungs-Sitzungsmanager (120) zur Registrierung bei diesem; und
Generieren und Speichern (638) eines Verknüpfungsbeziehungsdatensatzes, der die erste externe Vorrichtung (130, 904) als eine vertrauenswürdige Entität identifiziert, die dazu dient, sich an Therapieoperationen zu beteiligen, die die IMD (103, 903) betreffen.

4. Verfahren (600A-600D) nach Anspruch 3, wobei das Einrichten der vertrauenswürdigen Zuordnung zwischen der zweiten externen Vorrichtung (104, 905) und der IMD (103, 903) das Durchführen der folgenden Handlungen in einer zweiten Sitzung anschließend an die erste Sitzung umfasst:
Herstellen (642) einer zweiten bidirektionalen Kurzstrecken-Kommunikationsverbindung zwischen der zweiten externen Vorrichtung (104, 905) und der IMD (103, 903) durch Aktivieren einer zweiten Auslösevorrichtung, die dazu konfiguriert ist, zu veranlassen, dass die IMD (103, 903) in einen zweiten ausgewählten Kommunikationsinitialisierungsmodus versetzt wird;
Erhalten (644) eines oder mehrerer Vertrauensmerkmale von der IMD (103, 903) durch die zweite externe Vorrichtung (104, 905), wobei das eine oder die mehreren Vertrauensmerkmale Daten umfassen, die der IMD (103, 903) eindeutig zugeordnet sind;
Bereitstellen (646) des einen oder der mehreren Vertrauensmerkmale an den Fernbetreuungs-Sitzungsmanager (120) zum Validieren anhand der gespeicherten Vertrauensmerkmale, die beim Fernbetreuungs-Sitzungsmanager (120) in der ersten Sitzung registriert wurden;
als Reaktion auf das Validieren des einen oder der mehreren in der zweiten Sitzung bereitgestellten Vertrauensmerkmale, Generieren und Speichern (648) eines Verknüpfungsbeziehungsdatensatzes, der die zweite externe Vorrichtung (104, 905) als eine vertrauenswürdige Entität identifiziert, die dazu dient, sich an Therapieoperationen zu beteiligen, die die IMD (103, 903) betreffen.

5. Verfahren (600A-600D) nach Anspruch 4, wobei das eine oder die mehreren Vertrauensmerkmale mindestens eines von einer Seriennummer der IMD (103, 903), einem Auslesewert einer eingebauten Echtzeituhr, RTC, der IMD (103, 903), einem Zeitstempel, der angibt, wann die IMD (103, 903) in den Patienten (102, 902) implantiert wurde, einem in der IMD (103, 903) gespeicherten Vorrichtungsschlüssel, einem Vorrichtungsbezeichner, der der IMD (103, 903) zugeordnet ist, ein oder mehrere in der IMD (103, 903) gespeicherte biometrische Patientendaten und einem oder mehrere Programmbezeichner, die den in der IMD (103, 903) gespeicherten Therapieprogrammierungsdaten zugeordnet sind, umfassen.

6. Verfahren (600A-600D) nach Anspruch 5, wobei die erste Sitzung von einer medizinischen Fachkraft (906) ausgeführt wird, die die erste externe Vorrichtung (130, 904) bedient, und die zweite Sitzung vom Patienten (102, 902) ausgeführt wird, der die zweite externe Vorrichtung (104, 905) bedient, während die medizinische Fachkraft (906) und der Patient (102, 902) an einer persönlichen Erstberatung hinsichtlich der Initialisierung mindestens einer von jeweils der ersten und der zweiten externen Vorrichtung (130, 904; 104, 905) teilnehmen, um die eine oder die mehreren Operationen der Fernbetreuungstherapie zu ermöglichen, die über die IMD (103, 903) vermittelt werden.

7. Verfahren (600A-600D) nach Anspruch 6, wobei das Schlüsselinfrastruktursystem (122) ein System für eine Infrastruktur öffentlicher Schlüssel, PKI, umfasst, das dazu konfiguriert ist, ein erstes Schlüsselpaar bereitzustellen, das einen ersten öffentlichen Schlüssel und einen ersten privaten Schlüssel enthält, wobei das erste Schlüsselpaar mindestens einer von der ersten externen Vorrichtung (130, 904) und der medizinischen Fachkraft (906) zugeordnet ist, und wobei ferner ein erstes Zertifikat für den öffentlichen Schlüssel als Reaktion auf eine erste Zertifikatssignierungsanforderung, CSR, generiert wird, die von der ersten externen Vorrichtung (130, 904) ausgeht.

8. Verfahren (600A-600D) nach Anspruch 7, wobei das PKI-System dazu konfiguriert ist, ein zweites Schlüsselpaar bereitzustellen, das einen zweiten öffentlichen Schlüssel und einen zweiten privaten Schlüssel enthält, wobei das zweite Schlüsselpaar mindestens einer von der zweiten externen Vorrichtung (104, 905) und dem Patienten (102, 902) zugeordnet ist, und wobei ferner ein zweites Zertifikat für den öffentlichen Schlüssel als Reaktion auf eine zweite Zertifikatssignierungsanforderung, CSR, generiert wird, die von der zweiten externen Vorrichtung (104, 905) ausgeht.

9. System (100) zum Ermöglichen einer Fernbetreuungstherapie für einen Patienten (102), wobei das System (100) Folgendes umfasst:
eine erste externe Vorrichtung (130), die einer medizinischen Fachkraft (138) zugeordnet ist;
eine zweite externe Vorrichtung (104), die dem Patienten (102) zugeordnet ist;
eine implantierbare medizinische Vorrichtung, IMD, (103), die in den Patienten (102) implantiert ist, wobei die IMD (103) eine Therapieanwendung unterstützt, die dazu konfiguriert ist, von mindestens einem von der medizinischen Fachkraft (138), die die erste externe Vorrichtung (130) bedient, und dem Patienten (102), der die zweite externe Vorrichtung (104) bedient, programmierbar zu sein; und
einen Fernbetreuungs-Sitzungsmanager (120), der eine erste vertrauenswürdige Zuordnung zwischen der ersten externen Vorrichtung (130) und der IMD (103) und eine zweite vertrauenswürdige Zuordnung zwischen der zweiten externen Vorrichtung (104) und der IMD (103) unterstützt,
wobei die erste externe Vorrichtung (130) zu Folgendem konfiguriert ist:
Registrieren beim Fernbetreuungs-Sitzungsmanager (120);
Erhalten eines ersten Satzes von sicheren Kommunikationsberechtigungsdaten unter Verwendung eines Schlüsselinfrastruktursystems (122) in Zuordnung zu dem Fernbetreuungs-Sitzungsmanager (120), wobei der erste Satz von sicheren Kommunikationsberechtigungsdaten dazu dient, sichere Kommunikationen mit der zweiten externen Vorrichtung (104) herzustellen; und
Generieren einer ersten Verknüpfungsanforderung an den Fernbetreuungs-Sitzungsmanager (120) in Bezug auf die erste vertrauenswürdige Zuordnung, die hergestellt wird, wenn sich die erste externe Vorrichtung (130) und der Patient (102) in der Nähe zueinander befinden, wobei die erste Verknüpfungsanforderung ein oder mehrere Vertrauensmerkmale der IMD (103) zur Registrierung und Speicherung beim Fernbetreuungs-Sitzungsmanager (120) enthält,
wobei die zweite externe Vorrichtung (104) zu Folgendem konfiguriert ist:
Registrieren beim Fernbetreuungs-Sitzungsmanager (120);
Generieren einer zweiten Verknüpfungsanforderung an den Fernbetreuungs-Sitzungsmanager (120) in Bezug auf die zweite vertrauenswürdige Zuordnung, die hergestellt wird, wenn sich die zweite externe Vorrichtung (104) und der Patient (102) in der Nähe zueinander befinden, wobei die zweite Verknüpfungsanforderung ein oder mehrere Vertrauensmerkmale der IMD (103) enthält, die zur Validierung durch den Fernbetreuungs-Sitzungsmanager (120) anhand der gespeicherten Vertrauensmerkmale bereitgestellt werden, die in der ersten Verknüpfungsanforderung von der ersten externen Vorrichtung (130) empfangen wurden; und
nach dem erfolgreichen Herstellen der zweiten vertrauenswürdigen Zuordnung, Erhalten eines zweiten Satzes von sicheren Kommunikationsberechtigungsdaten unter Verwendung des Schlüsselinfrastruktursystems (122) in Zuordnung zu dem Fernbetreuungs-Sitzungsmanager (120), wobei der zweite Satz von sicheren Kommunikationsberechtigungsdaten dazu dient, sichere Kommunikationen mit der ersten externen Vorrichtung (130) herzustellen, und
wobei mindestens eine von der ersten und der zweiten externen Vorrichtung (130, 104) dazu dient, basierend auf der vertrauenswürdigen Zuordnung zwischen der ersten externen Vorrichtung und der IMD und der vertrauenswürdigen Zuordnung zwischen der IMD und der zweiten externen Vorrichtung einen sicheren Kommunikationskanal aufzubauen, um eine oder mehrere Operationen der Fernbetreuungstherapie in Bezug auf den Patienten (102) auszuführen, wobei der sichere Kommunikationskanal über eine Fernkommunikationsverbindung zwischen der ersten und der zweiten externen Vorrichtung (130, 104) hergestellt wird, wenn sich die erste externe Vorrichtung (130) entfernt von der zweiten externen Vorrichtung (104) befindet und sich die zweite externe Vorrichtung (104) in der Nähe des Patienten (102) befindet, wobei die eine oder die mehreren Operationen der Fernbetreuungstherapie zwischen der ersten externen Vorrichtung (130) und der IMD (103) des Patienten (102) vermittelt werden.

10. System (100) nach Anspruch 9, wobei der Fernbetreuungs-Sitzungsmanager (120) dazu konfiguriert ist, die erste externe Vorrichtung (130) mit einem Managementsystem für mobile Vorrichtungen für Unternehmen (307) in Zuordnung zum Registrieren der ersten externen Vorrichtung (130) zu validieren.

11. System (100) nach Anspruch 10, wobei das eine oder die mehreren Vertrauensmerkmale mindestens eines von einer Seriennummer der IMD (103), einem Auslesewert einer eingebauten Echtzeituhr, RTC, der IMD (103), einem Zeitstempel, der angibt, wann die IMD (103) in den Patienten (102) implantiert wurde, einem in der IMD (103) gespeicherten Vorrichtungsschlüssel, einem Vorrichtungsbezeichner, der der IMD (103) zugeordnet ist, ein oder mehrere in der IMD (103) gespeicherte biometrische Patientendaten und einem oder mehrere Programmbezeichner, die den in der IMD (103) gespeicherten Therapieprogrammierungsdaten zugeordnet sind, umfassen.

12. System (100) nach Anspruch 11, wobei die erste Verknüpfungsanforderung von der medizinischen Fachkraft (138) ausgeführt wird, die die erste externe Vorrichtung (130) bedient, und die zweite Verknüpfungsanforderung vom Patienten (102) ausgeführt wird, der die zweite externe Vorrichtung (104) bedient, während die medizinische Fachkraft (138) und der Patient (102) an einer persönlichen Erstberatung hinsichtlich der Initialisierung mindestens einer von jeweils der ersten und der zweiten externen Vorrichtung (130, 104) teilnehmen, um die Operationen der Fernbetreuungstherapie zu ermöglichen, die über die IMD (103) vermittelt werden.

13. System (100) nach Anspruch 9, wobei die IMD (103) dazu konfiguriert ist, mindestens eine von einer Therapie zur Rückenmarksstimulation, SCS, einer Therapie zur neuromuskulären Stimulation, einer Therapie zur Stimulation des Dorsalwurzelganglions, DRG, einer Therapie zur tiefen Hirnstimulation, DBS, einer Therapie zur Ohrmuschelstimulation, einer Medikamentenabgabetherapie, einer Herzschrittmachertherapie, einer Kardioverter-Defibrillator-Therapie, einer Therapie zum Herzrhythmusmanagement, CRM, einer elektrophysiologischen, EP, Kartierungs- und Hochfrequenz-, HF-, Ablationstherapie, einer Elektrokonvulsionstherapie, ECT, einer Therapie zur repetitiven transkraniellen Magnetstimulation, rTMS, einer Therapie zur Vagusnervstimulation, VNS, und einer oder mehreren Anwendungen zur Überwachung des physiologischen Zustands zu unterstützen.

## Revendications

1. Procédé (600A) permettant de faciliter une thérapie de soins à distance pour un patient (102, 902) ayant un dispositif médical implantable, IMD (103, 903), le procédé (600A) comprenant :
l'enregistrement (602) d'un premier dispositif externe (130, 904) auprès d'un gestionnaire de sessions de soins à distance (120) disposé dans un réseau (118) ;
l'obtention (604) de justificatifs d'identité de communication sécurisés pour le premier dispositif externe (130, 904) à l'aide d'un système d'infrastructure à clés (122) ;
l'établissement (606) d'une association de confiance entre le premier dispositif externe (130, 904) et l'IMD (103, 903) au niveau du gestionnaire de sessions de soins à distance (120) lorsque le premier dispositif externe (130, 904) et le patient (102, 902) sont à proximité l'un de l'autre ;
l'établissement (608) d'une association de confiance entre l'IMD (103, 903) et un second dispositif externe (104, 905) au niveau du gestionnaire de sessions de soins à distance (120) lorsque le second dispositif externe (104, 905) et le patient (102, 902) sont à proximité l'un de l'autre ;
l'obtention (610) de justificatifs d'identité de communication sécurisés pour le second dispositif externe (104, 905) à l'aide du système d'infrastructure à clés (122) ; et
le lancement (612), sur la base de l'association de confiance entre le premier dispositif externe et l'IMD et de l'association de confiance entre l'IMD et le second dispositif externe, d'un canal de communication sécurisé entre les premier et second dispositifs externes (130, 904 ; 104, 905) pour effectuer une ou plusieurs opérations de thérapie de soins à distance par rapport au patient (102, 902), dans lequel le premier dispositif externe (130, 904) est éloigné du second dispositif externe (104, 905) et le second dispositif externe (104, 905) est à proximité du patient (102, 902), la ou les opérations de thérapie de soins à distance étant médiées entre le premier dispositif externe (130, 904) et l'IMD (103, 903) du patient (102, 902).

2. Procédé (600A, 600B) selon la revendication 1, comprenant en outre la validation (622) du premier dispositif externe (130, 904) avec un système de gestion de dispositif mobile d'entreprise (307) en association avec l'enregistrement du premier dispositif externe (130, 904).

3. Procédé (600A-600C) selon la revendication 2, dans lequel l'établissement de l'association de confiance entre le premier dispositif externe (130, 904) et l'IMD (103, 903) comprend l'exécution des actions suivantes dans une première session :
l'effectuation (632) d'une première liaison de communication bidirectionnelle à courte portée entre le premier dispositif externe (130, 904) et l'IMD (103, 903) en activant un premier dispositif de déclenchement configuré pour amener l'IMD (103, 903) à être placé dans un premier mode d'initialisation de communication sélectionné ;
l'obtention (634) d'un ou plusieurs indices de confiance à partir de l'IMD (103, 903) par le premier dispositif externe (130, 904), le ou les indices de confiance comprenant des données associées de manière unique à l'IMD (103, 903) ;
la fourniture (636) du ou des indices de confiance au gestionnaire de sessions de soins à distance (120) pour enregistrement auprès de celui-ci ; et
la génération et le stockage (638) d'un enregistrement de relation de liaison identifiant le premier dispositif externe (130, 904) en tant qu'entité de confiance opérationnelle pour s'engager dans des opérations thérapeutiques impliquant l'IMD (103, 903).

4. Procédé (600A-600D) selon la revendication 3, dans lequel l'établissement de l'association de confiance entre le second dispositif externe (104, 905) et l'IMD (103, 903) comprend l'exécution des actions suivantes dans une seconde session postérieure à la première session :
l'effectuation (642) d'une seconde liaison de communication bidirectionnelle à courte portée entre le second dispositif externe (104, 905) et l'IMD (103, 903) en activant un second dispositif de déclenchement configuré pour amener l'IMD (103, 903) à être placé dans un second mode d'initialisation de communication sélectionné ;
l'obtention (644) d'un ou plusieurs indices de confiance à partir de l'IMD (103, 903) par le second dispositif externe (104, 905), le ou les indices de confiance comprenant des données associées de manière unique à l'IMD (103, 903) ;
la fourniture (646) du ou des indices de confiance au gestionnaire de sessions de soins à distance (120) pour validation par rapport aux indices de confiance stockés enregistrés au niveau du gestionnaire de sessions de soins à distance (120) dans la première session ;
en réponse à la validation du ou des indices de confiance fournis lors de la seconde session, la génération et le stockage (648) d'un enregistrement de relation de liaison identifiant le second dispositif externe (104, 905) en tant qu'entité de confiance opérationnelle pour s'engager dans des opérations thérapeutiques impliquant l'IMD (103, 903).

5. Procédé (600A-600D) selon la revendication 4, dans lequel le ou les indices de confiance comprennent au moins l'un parmi un numéro de série de l'IMD (103, 903), une lecture d'une horloge en temps réel, RTC, embarquée de l'IMD (103, 903), un horodatage indiquant le moment où l'IMD (103, 903) est implanté chez le patient (102, 902), une clé de dispositif stockée dans l'IMD (103, 903), un identificateur de dispositif associé à l'IMD (103, 903), une ou plusieurs données biométriques de patient stockées dans l'IMD (103, 903), et un ou plusieurs identificateurs de programme associés à des données de programmation thérapeutique stockées dans l'IMD (103, 903).

6. Procédé (600A-600D) selon la revendication 5, dans lequel la première session est effectuée par un clinicien (906) mettant en œuvre le premier dispositif externe (130, 904) et la seconde session est effectuée par le patient (102, 902) mettant en œuvre le second dispositif externe (104, 905) lorsque le clinicien (906) et le patient (102, 902) sont engagés dans une consultation initiale en personne en ce qui concerne l'initialisation d'au moins l'un parmi de premier et second dispositifs externes respectifs (130, 904 ; 104, 905) pour faciliter la ou les opérations de thérapie de soins à distance médiées par l'intermédiaire de l'IMD (103, 903).

7. Procédé (600A-600D) selon la revendication 6, dans lequel le système d'infrastructure à clés (122) comprend un système d'infrastructure à clés publiques, PKI, configuré pour fournir une première paire de clés comportant une première clé publique et une première clé privée, la première paire de clés étant associée à au moins l'un parmi le premier dispositif externe (130, 904) et le clinicien (906), et en outre dans lequel un premier certificat de clé publique est généré en réponse à une première demande de signature de certificat, CSR, émanant du premier dispositif externe (130, 904).

8. Procédé (600A-600D) selon la revendication 7, dans lequel le système PKI est configuré pour fournir une seconde paire de clés comportant une seconde clé publique et une seconde clé privée, la seconde paire de clés étant associée à au moins l'un parmi le second dispositif externe (104, 905) et le patient (102, 902), et en outre dans lequel un second certificat de clé publique est généré en réponse à une seconde demande de signature de certificat, CSR, émanant du second dispositif externe (104, 905).

9. Système (100) permettant de faciliter une thérapie de soins à distance pour un patient (102), le système (100) comprenant :
un premier dispositif externe (130) associé à un clinicien (138) ;
un second dispositif externe (104) associé au patient (102) ;
un dispositif médical implantable, IMD, (103) implanté chez le patient (102), l'IMD (103) prenant en charge une application thérapeutique configurée pour être programmable par au moins l'un parmi le clinicien (138) mettant en œuvre le premier dispositif externe (130) et/ou le patient (102) mettant en œuvre le second dispositif externe (104) ; et
un gestionnaire de sessions de soins à distance (120) prenant en charge une première association de confiance entre le premier dispositif externe (130) et l'IMD (103) et une seconde association de confiance entre le second dispositif externe (104) et l'IMD (103),
dans lequel le premier dispositif externe (130) est configuré pour :
s'enregistrer auprès du gestionnaire de sessions de soins à distance (120) ;
obtenir un premier ensemble de justificatifs d'identité de communication sécurisés à l'aide d'un système d'infrastructure à clés (122) en association avec le gestionnaire de sessions de soins à distance (120), le premier ensemble de justificatifs d'identité de communication sécurisés étant opérationnel pour effectuer des communications sécurisées avec le second dispositif externe (104) ; et
générer une première demande de liaison au gestionnaire de sessions de soins à distance (120) par rapport à la première association de confiance effectuée lorsque le premier dispositif externe (130) et le patient (102) sont à proximité l'un de l'autre, dans lequel la première demande de liaison comporte un ou plusieurs indices de confiance de l'IMD (103) pour l'enregistrement et le stockage au niveau du gestionnaire de sessions de soins à distance (120),
dans lequel le second dispositif externe (104) est configuré pour :
s'enregistrer auprès du gestionnaire de sessions de soins à distance (120) ;
générer une seconde demande de liaison au gestionnaire de sessions de soins à distance (120) par rapport à la seconde association de confiance effectuée lorsque le second dispositif externe (104) et le patient (102) sont à proximité l'un de l'autre, dans lequel la seconde demande de liaison comporte un ou plusieurs indices de confiance de l'IMD (103) fournis pour validation au niveau du gestionnaire de sessions de soins à distance (120) par rapport aux indices de confiance stockés reçus dans la première demande de liaison à partir du premier dispositif externe (130) ; et
après avoir effectué avec succès la seconde association de confiance, obtenir un second ensemble de justificatifs d'identité de communication sécurisés à l'aide du système d'infrastructure à clés (122) en association avec le gestionnaire de sessions de soins à distance (120), le second ensemble de justificatifs d'identité de communication sécurisés étant opérationnel pour effectuer des communications sécurisées avec le premier dispositif externe (130), et
dans lequel au moins l'un des premier et second dispositifs externes (130, 104) est opérationnel pour lancer, sur la base de l'association de confiance entre le premier dispositif externe et l'IMD et de l'association de confiance entre l'IMD et le second dispositif externe, un canal de communication sécurisé pour effectuer une ou plusieurs opérations de thérapie de soins à distance par rapport au patient (102), le canal de communication sécurisé étant effectué par l'intermédiaire d'une connexion de communication à distance entre les premier et second dispositifs externes (130, 104) lorsque le premier dispositif externe (130) est éloigné du second dispositif externe (104) et le second dispositif externe (104) est à proximité du patient (102), la ou les opérations de thérapie de soins à distance étant médiées entre le premier dispositif externe (130) et l'IMD (103) du patient (102).

10. Système (100) selon la revendication 9, dans lequel le gestionnaire de sessions de soins à distance (120) est configuré pour valider le premier dispositif externe (130) avec un système de gestion de dispositif mobile d'entreprise (307) en association avec l'enregistrement du premier dispositif externe (130).

11. Système (100) selon la revendication 10, dans lequel le ou les indices de confiance comprennent au moins l'un parmi un numéro de série de l'IMD (103), une lecture d'une horloge en temps réel, RTC, embarquée de l'IMD (103), un horodatage indiquant le moment où l'IMD (103) est implanté chez le patient (102), une clé de dispositif stockée dans l'IMD (103), un identificateur de dispositif associé à l'IMD. (103), une ou plusieurs données biométriques de patient stockées dans l'IMD (103), et un ou plusieurs identificateurs de programme associés à des données de programmation thérapeutique stockées dans l'IMD (103).

12. Système (100) selon la revendication 11, dans lequel la première demande de liaison est effectuée par le clinicien (138) mettant en œuvre le premier dispositif externe (130) et la seconde demande de liaison est effectuée par le patient (102) mettant en œuvre le second dispositif externe (104) lorsque le clinicien (138) et le patient (102) sont engagés dans une consultation initiale en personne en ce qui concerne l'initialisation d'au moins l'un parmi de premier et second dispositifs externes respectifs (130, 104) pour faciliter les opérations de thérapie de soins à distance médiées par l'intermédiaire de l'IMD (103).

13. Système (100) selon la revendication 9, dans lequel l'IMD (103) est configuré pour prendre en charge au moins l'une parmi une thérapie de stimulation de la moelle épinière, SCS, une thérapie de stimulation neuromusculaire, une thérapie de stimulation du ganglion de la racine dorsale, DRG, une thérapie de stimulation cérébrale profonde, DBS, une thérapie de stimulation cochléaire, une thérapie d'administration de médicaments, une thérapie de stimulation cardiaque par stimulateur cardiaque, une thérapie de stimulation cardiaque par défibrillateur-cardioverteur, une thérapie de gestion du rythme cardiaque, CRM, une thérapie de cartographie électrophysiologique, EP, et d'ablation par radiofréquence, RF, une thérapie par électroconvulsions, ECT, une thérapie de stimulation magnétique transcrânienne répétitive, rTMS, une thérapie de stimulation du nerf vague, VNS, et une ou plusieurs applications de surveillance de l'état physiologique.
